Europäisches Patentamt

European Patent Office

Office européen des brevets

(11) Publication number : **0 478 366 A2**

# EUROPEAN PATENT APPLICATION

(21) Application number : 91308855.5

(22) Date of filing : 27.09.91

(51) Int. Cl.⁵ : **C12N 15/62**, C12P 21/08,
A61K 39/395, A61K 49/02,
// C12N9/72

(30) Priority : 27.09.90 US 589435

(43) Date of publication of application :
01.04.92 Bulletin 92/14

(84) Designated Contracting States :
AT BE CH DE DK ES FR GB GR IT LI LU NL SE

(71) Applicant : **THE GENERAL HOSPITAL
CORPORATION**
**55 Fruit Street**
**Boston, MA 02114 (US)**

(72) Inventor : **Haber, Edgar**
**83 Ridgeway Road**
**Weston, Massachusetts 02193 (US)**
Inventor : **Quertermous, Thomas**
**240 Brattle Street, Apt. 41**
**Cambridge, Massachusetts 02138 (US)**

(74) Representative : **Sheard, Andrew Gregory et al**
**Kilburn & Strode 30, John Street**
**London WC1N 2DD (GB)**

(54) Recombinant hybrid immunoglobulin molecules and method of use.

(57)    A recombinant chimeric immunoglobulin molecule is disclosed which has an antigen binding site specific for fibrin linked to the active portion of a plasminogen activator, such as urokinase.

EP 0 478 366 A2

This invention relates to a recombinant hybrid immunoglobulin molecule having an antigen binding site specific for fibrin linked to a second protein comprising the active portion of a plasminogen activator. This invention also is directed to the cloning and production of these novel hybrid immunoglobulin molecules. This invention further relates to a method of using these hybrid immunoglobulin molecules in immunodiagnostic and immunotherapeutic processes.

Most myocardial infarctions are caused by coronary thrombosis (DeWood et al., N. Eng. J. Med., 303:897 (1983)). The coronary thrombosis that causes the myocardial infarction can be lysed by thrombolytic agents. These thrombolytic agents are plasminogen activators that activate the conversion of plasminogen to the fibrinolytic enzyme plasmin. Plasmin will then lyse the fibrin present in the thrombus. This treatment with plasminogen activators is not without side effects. Plasmin acts non-selectively and therefore, not only lyses the fibrin in the thrombus, but also attacks fibrinogen and clotting factors, often resulting in severe bleeding diathesis.

Streptokinase, urokinase, prourokinase, and tissue-type plasminogen activator (tPA) are known plasminogen activators for lysing thrombi. These activators are indicated for the treatment for acute cardiovascular disease such as infarct, stroke, pulmonary embolism, deep vein thrombosis, peripheral arterial occlusion, and other venous thrombosis. Both streptokinase and urokinase, however, have severe limitations. Due to a low affinity for fibrin, both activators will activate circulating and fibrin-bound plasminogen indiscriminately. The plasmin formed in circulating blood is neutralized before it can be used in thrombolysis. Residual plasmin will degrade several clotting factor proteins, for example, fibrinogen, factor V, and factor VIII, causing hemorrhagic potential. Further, streptokinase is strongly antigenic and patients with high antibody titers respond inefficiently to treatment and cannot remain on continuous treatment.

Human tissue-type plasminogen activator can bind to fibrin and therefore favors the activation of plasminogen in close proximity to the thrombus, potentially sparing fibrinogen elsewhere in the circulation. However, at doses required for prompt lysis of coronary thrombi, the use of tissue-type plasminogen activator can also result in hemorrhage.

In order to increase the specificity of the thrombolytic agents to the thrombus, it has been shown that covalent linkage of urokinase to a fibrin-specific antibody results in marked enhancement of fibrinolytic potency and specificity. Bode et al., Science 229:765-767 (1985).

One function characteristic of every antibody molecule is specific binding to an antigenic determinant. Antibodies in vivo are bivalent and monospecific, containing two identical antigen binding sites. The specific binding of antigen by an antibody molecule is determined by the antibody's structure of the variable regions ($F_{ab}$) of both heavy and light chains.

Antibodies having dual specificities have been prepared by subjecting antibodies of different specificities to a selective cleavage of the disulfide bridges that link the two heavy chains together. Antibody half-molecules are then reassociated under neutral pH to produce the hybrid antibodies having dual specificities. See Nisonhoff et al., Nature(London) 394:355 (1962); Brennan et al., Science 229:31 (1985); Liu et al., Proc. Natl. Acad. Sci. USA 82:8648 (1985); and commonly assigned co-pending United States Patent Application, Serial No. 851,554, filed April 14, 1986.

Bispecific antibodies have also been produced from hybridomas. The preparation of bispecific monoclonal antibodies by fusion of antibody-producing hybridoma cells is described in Milstein and Cuello, Nature [London] 305:537 (1983) and in PCT application, WO83 103679.

Antibodies have also been cloned and produced by recombinant DNA techniques. Genes for heavy and light chains have been introduced into appropriate hosts and expressed, followed by reaggregation of these individual chains into functional antibody molecules (see for example Munro, Nature 312:597 (3984); Morrison, S.L. Science 229:1202 (1985]; Oi et al., BioTechniques 4:214 (1986)); Wood et al., Nature 314U:446-[449] (1985)). Light and heavy chain variable regions have been cloned and expressed in foreign hosts, and maintain their binding ability (Moore et al., European Patent Publication 0088994 (published September 21, 1983)).

Chimeric or hybrid antibodies have also been prepared by recombinant DNA techniques. Oi and Morrison, BioTechniques 4:214 (1986), describe a strategy for producing chimeric antibodies. On pages 218-220 a chimeric:human IgG anti-Leu[3] antibody is described. The authors state that a chimeric mouse:human anti-dansyl antibody has been made. This article indicates, without specifically stating, that the Leu3 binding specificity and the anti-dansyl binding specificity have been cloned together into a single immunoglobulin molecule.

Morrison, Science 229:1202 (1985), in Table 1, states that variable light or variable heavy chain regions can be attached to a non-Ig sequence to create fusion proteins. This article states that the potential uses for the fusion proteins are three: (1) to attach antibody specificity to enzymes for use in assays; (2) to isolate non-Ig proteins by antigen columns; and (3) to specifically deliver toxic agents. There is no description in this reference as to any specific chimeric immunoglobulin molecule.

Neuberger et al., Nature 314:268 (1985), describes a chimeric antibody whose heavy chain is a human constant region fused to a mouse variable region that is specific for the hapten, 4-hydroxy-3-nitrophenyl-acetyl.

European Patent Application 120,694 describes the genetic engineering of the variable and constant regions of an immunoglobulin molecule that is expressed in E. coli host cells. On page 10 of the application, it states that the immunoglobulin molecule may be synthesized by a host cell with another peptide moiety attached to one of the constant domains. This peptide moiety is described as either cytotoxic or enzymatic. It also states on page 10 that the immunoglobulin molecule may also comprise a therapeutic agent. The description in the application and in the examples describe the use of a lamda-like chain derived from a monoclonal antibody which binds to 4-hydroxy-3-nitropenylacetal (NP) haptens.

European Patent Application 125,023 relates to the use of recombinant DNA techniques to produce immunoglobulin molecules that are chimeric or otherwise modified. One of the uses described in pages 3-4 for these immunoglobulin molecules is the use of whole body diagnosis and treatment by injecting the antibodies, directed to specific target disease tissues, into a patient. The presence of the disease can be determined by attaching a suitable label to the antibodies, or the diseased tissue can be attacked by carrying a suitable drug with antibodies. The application describes antibodies engineered to aid the specific delivery of an agent as "altered antibodies." PCT application W083/03971 relates to a hybrid protein that comprises antibody-enzymatically active toxins.

PCT application W083/01533 describes on page 5 chimeric antibodies with the variable region of an immunoglobulin molecule linked to a portion of a second protein which may comprise the active portion of an enzyme.

Boulianne et al., Nature 312:643 (1984) constructed an immunoglobulin gene in which the DNA segments that encode mouse variable regions specific for the hapten trinitrophenol are joined to segments that encode human mu and kappa constant regions. These chimeric genes were expressed as functional TNP-binding chimeric IgM.

Morrison et al., Proc. Natl. Acad. Sci. USA 81:6851 (1984) created a chimeric molecule utilizing the heavy chain variable region axons of an anti-phosphoryl choline myeloma protein gene, which were joined to the axons of either human kappa light chain gene. The genes were transfected into mouse myeloma cell lines, generating transformed cells that produced chimeric mouse-human ZgG with antigen binding function.

Sharon et al., Nature 309:604 (1984), fused a gene encoding a mouse heavy chain variable region specific for azophenylarsonate with the mouse kappa light chain constant region gene. This construct resulted in a polypeptide chain that dimerized with the corresponding $V_L$-Kappa polypeptide chain when introduced into the appropriate myeloma cell line. The $V_{Hkappa}V_LC_{kappa}$ molecule was bound to the azophenyl-arsonate hapten.

Neuberger et al., Nature 312:604 (1984) joined the heavy chain variable region gene of a hapten-specific antibody to a gene specifying the synthesis of micrococcal nuclease, and obtained a hybrid molecule that had both antigen binding and enzymatic activity.

It would be desirable to have a selective plasminogen activator that is characterized by high affinity and specificity for fibrin relative to fibrinogen, and that would effect activation of plasminogen only in the immediate environment of a fibrin-containing thrombus.

Thus a first aspect of the present invention relates to a (e.g. chimeric) immunoglobulin molecule comprising:

an antibody variable region with an antigen binding site specific for fibrin; and

a fibrinolytic enzyme activity region.

Preferably the immunoglobulin molecule is recombinant.

Suitably the fibrinolytic enzyme is tissue-type plasminogen activator, streptokinase, urokinase or prourokinase. It is preferred that the fibrinolytic enzyme is single chain urokinase.

Advantageously the antibody variable region is from antibody 59D8. Thus in preferred embodiments the immunoglobulin molecule is r-scuPA(32)-59D8.

The invention at its broadest therefore relates to an (e.g. recombinant hybrid) immunoglobulin molecule having an antibody variable region with an antigen binding site specific for fibrin and a fibrinolytic enzyme activity region.

A second aspect of the present invention relates to nucleic acid encoding for the immunoglobulin of the first aspect. The nuclei acid will suitable be a recombinant DNA molecule. Preferably the molecule will comprise the 3′ untranslated region of β-globin. It may also suitably comprise the 3′ untranslated region of lambda2b heavy chain gene.

Thus the nucleic acid (or recombinant DNA molecule) may comprise pSVUKG(Ig). It may also comprise pSVUKG(β).

A third aspect of the present invention relates to a vector (such as an expression vector) comprising the nucleic acid (or DNA molecule) of the second aspect.

A fourth aspect of the present invention relates to a host (e.g. cell) comprising the vector of the third aspect (which will suitable be an expression vector ) .

A fifth aspect of the present invention relates to a process for the preparation of an (e.g. chimeric) immunoglobulin molecule of the first aspect, the process comprising:

transforming a host (e.g. cell) with an expression vector of the third aspect;

expressing nucleic acid encoding for the immunoglobulin molecule, for example by growing the host (cell) such that nucleic acid is expressed; and

optionally purifying the (e.g. chimeric) immunoglobulin molecule.

A sixth aspect of the present invention relates to a pharmaceutical composition comprising an immunoglobulin molecule of the first aspect and a pharmaceutically acceptable carrier.

The invention in its seventh aspect contemplates a method of detecting a thrombus, the method comprising:

(a) administering to a host an immunoglobulin molecule of the first aspect which is labelled with a detectable (or selectable) marker, such as a radiolabel and/or paramagnetic isotope; and

(b) detecting the presence of a thrombus.

In an eighth aspect the invention relates to the immunoglobulin molecule of the first aspect for use in medicine.

Thus a ninth aspect of the present invention contemplates the use of the immunoglobulin molecule of the first aspect in the preparation of an agent for treating a thrombus and/or a thrombolytic, fibrinolytic or diagnostic agent.

A tenth aspect of the present invention relates to a process for the preparation of nucleic acid (such as a recombinant DNA molecule) of the second aspect, the process comprising coupling successive nucleotides and/or oligo- or poly-nucleotides together.

An eleventh aspect relates to a process for the preparation of a vector of the third aspect, the process comprising coupling successive nucleotides and/or oligo- or poly-nucleotides together,

A twelfth aspect of the present invention relates to a process for the preparation of a host of the fourth aspect, the process comprising transforming or transfecting a host (e.g. cell) with a vector of the third aspect or as prepared in accordance with the eleventh aspect.

The invention in its thirteenth aspect relates to a process for the preparation of a pharmaceutical composition, the process comprising admixing an immunoglobulin molecule of the first aspect with a pharmaceutically acceptable carrier.

Preferred features and characteristics of one aspect of the present invention are as for another aspect *mutatis mutandis*.

The invention thus relates to a recombinant hybrid (e.g. chimeric) immunoglobulin molecule having an antigen binding site specific for fibrin linked to a second protein comprising the active portion of a plasminogen activator. The invention also relates to the cloning and production of these novel hybrid immunoglobulin molecules. The invention further contemplates a method of using these recombinant hybrid immunoglobulin molecules in immunodiagnostic and immunotherapeutic processes.

The invention also encompasses genetic sequences coding for the hybrid immunoglobulin molecules, cloning and expression vectors containing such genetic sequences, hosts transformed with such vectors, and methods for producing such hybrid molecules by expression of the underlying genetic sequences in such hosts.

The invention at its broadest is directed to a hybrid immunoglobulin molecule that has both antigen binding site and enzyme activity. More specifically the invention is directed to a recombinant hybrid immunoglobulin molecule having an antigen binding site specific for fibrin linked to a second protein comprising the active portion of plasminogen activator. The invention also encompasses cloning and production of these novel hybrid immunoglobulin molecules.

Throughout this specification, the term "hybrid immunoglobulin molecule" is used to designate a single molecule produced by recombinant DNA techniques that comprises all, or a portion of, a fibrin-specific antibody and all, or a portion of, a plasminogen activator. Heterobifunctional antibody, heteroantibody, bispecific antibody, heteroligating antibody, antibody duplex, and heterodimer are all terms that refer more specifically to an antibody of dual specificity, that is, two antibody combining sites in one molecule.

Fibrin specificity as used herein refers to antibodies raised against fibrin. When blood escapes from the vasculature, an intricate cascade of enzymatic reactions converts fibrinogen to fibrin, the structural protein in clotted blood. Fibrinogen itself is the least soluble of the plasma proteins. With a 340,000 kd MW, it possesses a two-fold symmetry arising from three pairs of nonidentical polypeptide chains called A-alpha B-beta, and gamma. At the site of thrombosis, the coagulation cascade is activated to generate thrombin, which enzymatically cleaves polar peptides (Fibrinopeptide A from A-alpha and Fibrinopeptide B from B-beta), and results in fibrin monomer formation. Fibrin monomers, being much less soluble, spontaneously polymerize into a gel network. After polymerization, the fibrin clot is stabilized by Factor XIIIa, which introduces covalent interchain e-(g-glutamyl)lysine bonds. Fibrinogen and fibrin are identical in greater than 98% of their structure and differ only in two newly exposed amino termini, those of the fibrin alpha and beta chains. The amino acid sequence of these fibrin amino termini is known. Doolittle, R.F., "Fibrinogen and Fibrin," in Putnam, F.W., ed., The Plasma Proteins: Structure, Function, and Genetic Control, 3d ed., Vol. 2, New York: Academic Press, 1975, pp. 109-156.

Fibrin epitopes that may be used in this invention include the amino terminus of the fibrin beta chain, the amino terminus of the fibrin alpha chain, the beta (43-49) amino acid sequences, which are carboxy-terminal to a plasmin cleavage site, and the gamma chain crosslink site.

Antibodies with specificity to fibrin have been described in Hui et al., Science 222:1129 (1983). Further description of the same type of antibodies can be found in commonly assigned co-pending United States Application, Serial No. 824,228, filed January 30, 1986, for "Fibrin-Specific Monoclonal Antibodies Lacking Fibrinogen Cross-Reactivity." Fibrin-specific monoclonal antibodies with substantially no fibrinogen cross-reactivity are also described in commonly assigned co-pending United States Patent Application Serial No. 851,514, filed April 14. 1986. Other examples of antibodies with a specificity against fibrin include Kudryk et al.,Mol. Imm. 21:89 (1984); European Patent Application 146,050 to Callewaert, published June 26, 1985, for "Site Selective plasminogen Activator and Method of Making and Using Same"; and Australian Patent Application, AV-A-25387/84 to Bundesen et al. for "Monoclonal Antibodies with Specificity for Crosslinked Fibrin and Their Diagnostic Uses."

In preparing the hybrid immunoglobulin molecules of this invention, the entire fibrin-specific antibody may be cloned and comprise a portion of the hybrid molecule. However, in order to reduce the size of the hybrid, immunoglobulin molecule, and to reduce antigenicity, it is preferred to use only that region of the antibody that will recognize and bind to fibrin.

Cloning this region of the fibrin-specific antibody requires an understanding of the structure and function of antibodies. Briefly, antibodies are tetrameric immunoglobulins consisting of two identical light (L) chains and two identical heavy (H) chains. Each protein chain consists of two principle regions: the N-terminal variable (V) region and the C-terminal constant (C) region. The variable light ($V_L$) and heavy ($V_H$) chains form the variable region domain. The variable domain determines recognition and specificity to a particular antigen. The constant region domains of light ($C_L$) and heavy ($C_H$) chains mediate the effector function responsible for executing the immune response. The hinge region (J) of the antibody molecule connects the Fab fragment to the Fc fragment of the antibody.

Within the variable region, there may be hypervariable regions known as diversity domains (D). These diversity domains are related to axons observed in the genes encoding for the variable regions.

The variable domain of an antibody, a protein structural definition, consists of both VL and VH segments of the light and heavy chains. It contains 6 hypervariable regions, three in the light chain and three in the heavy chain. On a genetic level, three axons are responsible for specifying VH, including its framework, and hypervariable regions; two axons specify VL. The first two hypervariable regions of both VL and VH are specified by the V gene axons of the light and heavy chains respectively. The third hypervariable region of the light chain is specified by two axons, VL and JL. The third hypervariable region of the heavy chain is specified by three axons vH, D, and JH.

Immunoglobulin gene expression occurs through the joining of the V gene to the G gene by somatic recombination in the B lymphocytes. These genes are joined to form the complete immunoglobulin. The rearranged, joined gene segments then encode the complete immunoglobulin or antigen binding domains of light and heavy variable chains.

There are five principal classes of heavy chains, characterized by chemical and isotopic properties. These heavy chain classes are referred to as mu, gamma delta, alpha, and epsilon. There are also two principal classes of light chains: kappa and lambda.

In this invention, an antibody specific for fibrin is cloned as part of the hybrid immunoglobulin molecule. Preferably, only the variable region of the fibrin antibody is cloned. Either the variable light or variable heavy chain, or both, comprises part of the hybrid molecule. In addition, the hinge region of the fibrin-specific antibody may be cloned. The constant domain of the Fab portion of the fibrin-specific antibody joined to the variable region may also be cloned. The variable and constant region of the fibrin-specific antibody cloned and used in the hybrid immunoglobulin molecule may be derived from a mammalian source, with the preferred source from humans. Alternatively, the variable region may be from a mammalian source, with the constant region from a human source.

Plasminogen activators that may be used in this invention include urokinase, prourokinase, tissue type plasminogen activator, and streptokinase. When plasminogen is converted by an activator to plasmin, the active fibrinolytic enzyme of plasma, it develops a marked affinity for its substrate, fibrin.

The term "plasminogen activator" is therefore a thrombolytic agent and is meant to include in this specification any agent utilized. Other terms are known in the art for the lysis of a thrombus, including fibrinolysis. Although the most common plasminogen activators are streptokinase, urokinase, prourokinase and tissue-type plasminogen activator, any other plasminogen activator or thrombolytic agent may be used in this invention.

In preparing the hybrid immunoglobulin molecules of this invention, the entire plasminogen activator may be cloned and expressed as part of the hybrid molecule. Preferably, only the active portion of the plasminogen activator is cloned. This active site or catalytic site may be determined by routine screening as described in the examples.

The process for obtaining a hybrid immunoglobulin molecule according to the present invention requires the cloning of the fibrin-specific antibody and the plasminogen activator and expression of their DNA sequences into a single hybrid molecule.

The DNA sequences of the fibrin-specific antibody and the plasminogen activator employed for preparation of the hybrid immunoglobulin molecule may be derived from a variety of sources. These sources include genomic DNA, cDNA, synthetic DNA, and combinations thereof. The genomic DNA may or may not include naturally occurring introns.

The DNA obtained from the genomic DNA or cDNA may be obtained in a variety of ways. Cells coding for the desired sequence may be isolated, the genomic DNA fragmented, conveniently by one or more restriction endonucleases, and the resulting fragments cloned and screened with a probe for the presence of the DNA sequence coding for fibrin-specificity or for the plasminogen activator.

For the variable region of the fibrin-specific antibody, the rearranged heavy chain coding DNA may include V, D, and J regions. The rearranged germline light chain coding DNA may include the V and J regions. Once the cloned fragment has been identified which contains the desired fibrin-specific DNA sequence binding site, this fragment may be further manipulated to remove superfluous DNA, modify one or both termini, remove all or a portion of intervening sequences (introns) or the like.

The joining of the various fragments is performed in accordance with conventional techniques, employing blunt-ended or staggered-ended termini for ligation, restriction enzyme digestion to provide for appropriate termini, filling in of cohesive ends as appropriate, alkaline phosphatase treatment to avoid undesirable joining, and ligation with appropriate ligases.

For cDNA, the cDNA may be cloned and the resulting clone screened with an appropriate probe for cDNA coding for the desired variable or constant region. Once the desired clone has been isolated, the cDNA may be manipulated in substantially the same manner as the genomic DNA. However, with cDNA there will be no introns or intervening sequences.

Further, the genes or the fibrin-specific antibody and the genes of the plasminogen *activator may* be synthesized according to well-known means and cloned for use in preparing the hybrid immunoglobulin molecule.

To express the hybrid immunoglobulin molecule, transcriptional and translational signals recognized by an appropriate host are necessary. Eukaryotic hosts will be mammalian cells capable of culture in vitro, particularly leukocytes. More particularly myeloma cells, or other transformed or oncogenic lymphocyte, e.g., EBV transformed cells. Alternatively, non-mammalian cells may be employed, such as bacteria, fungi, e.g., yeast, filamentous fungi, or the like.

The DNA sequence coding for the fibrin-specific variable region may be obtained in association with the promoter region from genomic DNA. To the extent that the host cells recognize the transcriptional regulatory and translational initiation signals associated with the variable region, then the region 5′ of the variable region coding sequence may be retained and employed for transcriptional and translational initiation regulation.

The contiguous non-coding region 5′ to the variable region will normally include those sequences involved with initiation of transcription and translation, such as the TATA box, capping sequence, CAAT sequence, and the like. Usually the 5′-non-coding sequence will be at least 150 bp, more usually at least 200 bp, usually not exceeding about 2k bp, more usually not exceeding about 1k bp.

The non-coding region 3′ to the fibrin specific constant region may be retained for its transcriptional termination regulatory sequences, such as termination and polyadenylation. In addition, the non-coding region 3′ to the coding region also contains an important enhancer in immunoglobulin genes. Thus, by retaining the 3′-region naturally contiguous to the DNA sequence coding for the constant region, the transcriptional termination signals may be provided. Where the transcriptional termination signals are not satisfactorily functional in the expression host cell, then a 3′ region functional in the host cell may be substituted.

The constructs for the fibrin-specific antibody and the plasminogen activator may be joined together to farm a single DNA segment or may be maintained as separate segments, by themselves or in conjunction with vectors.

The construct(s) may be introduced into a cell by transformation in conjunction with a gene allowing for selection where the construct will become integrated into the host genome. Usually the construct will be part of a vector having a replication system recognized by the host cell.

Expression vehicles for production of the molecules of the invention include plasmids or other vectors. In general, such vectors containing replicon and control sequences which are derived from species compatible with a host cell are used in connection with the host. The vector ordinarily carries a replicon site, as well as specific genes which are capable of providing phenotypic selection in transformed cells. For example, E. coli is readily transformed using pBR322, a plasmid derived from an E. coli species. pBR322 contains genes for ampicillin and tetracycline resistance, and thus provides easy means for identifying transformed cells. The pBR322 plasmid or other microbial plasmids must also contain, or be modified to contain, promoters which can be used by the microbial organism for expression of its own proteins. Those promoters most commonly used in recombinant DNA construction include the beta lactamase, lactose promoter systems, lambda phage promoters, and the tryptophan promoter systems. While these are the most commonly used, other microbial promoters have been discovered and can be utilized.

For example, a genetic construct for the hybrid immunoglobulin molecule can be placed under the control of the leftward promoter of bacteriophage lambda. Control is exerted by the lambda repressor, and adjacent restriction sites are known.

The expression of the hybrid immunoglobulin molecule can also be placed under control of other regulatory sequences which may be homologous to the organism in its untransformed state. For example, lactose dependent E. coli chromosomal DNA comprises a lactose or lac operon which mediates lactose utilization by elaborating the enzyme beta-galactosidase. The lac control elements may be obtained from bacteriophage lambda plac5, which is ineffective for E. coli. The lac promoter-operator system can be induced by IPTG.

The expression of the hybrid immunoglobulin molecule can be optimized by replacing the 3′ untranslated region in the plasmid with the 3′ untranslated region of either beta globin or mouse immunoglobulin. For the most part, the 3′ untranslated region which is replaced is that of the plasminogen activator.

Other promoter/operator systems or portions thereof can be employed as well. For example, colicin E1, galactose, alkaline phosphatase, tryptophan xylose, tax, and the like can be used.

The preferred hosts are mammalian cells, grown in vitro in tissue culture, or in vivo in animals. Mammalian cells proride post translational modifications to immunoglobulin protein molecules including correct folding or glycosylation at correct sites.

Mammalian cells which may be useful as hosts include cells of fibroblast origin such as VERO or CHO-K$_1$ or cells of lymphoid origin, such as the hybridoma SP2/0-AG14 or the myeloma P3x63Sg8, and their derivatives. Preferred mammalian host cells include SP2/0 and J558L. Several cell lines secrete urokinase and may be used for transfection, such as cultured kidney carcinoma cells (Ferraivolo et al., J. Cell. Physiol. 121:363 (1984)) and 3T3 cells (Belin et al., EMBO J. 3:390 (1984)).

For a mammalian host, several possible vector systems are available for the expression of the hybrid immunoglobulin molecule. One class of vectors utilizes DNA elements which provide autonomously replicating extra-chromosomal plasmids, derived from animal viruses such as bovine papilloma virus, polyoma virus, adenovirus, or SV40 virus. A second class of vectors relies upon the integration of the desired gene sequences into the host cell chromosome. Cells which have stably integrated the introduced DNA into their chromosomes can be selected by also introducing one or more markers which allow for selection of host cells which contain the expression vector. The marker may provide for prototrophy to an auxotropic host, biocide resistance, e.g., antibiotics, or heavy metals, such as copper, or the like. The selectable marker gene can either be directly linked to the DNA gene sequences to be expressed, or introduced into the same cell by co-transfection. Additional elements may also be needed for optimal synthesis of single chain binding protein mRNA. These elements may include splice signals, as well as transcription promoters, enhancers, and termination signals. cDNA expression vectors incorporating such elements include those described by Okayama, H., Mol. Cel. Biol. 3:280 (1983), and others.

A wide variety of transcriptional and translational regulatory sequences may be employed, depending upon the nature of the host. The transcriptional and translational regulatory signals may be derived from viral sources, such as adenovirus, bovine papilloma virus, Simian virus, or the like, where the regulatory signals are associated with a particular gene which has a high level of expression. Alternatively, promoters from mammalian expression products, such as actin, collagen, myosin, etc., may be employed. Transcriptional initiation regulatory signals may be selected which allow for repression or activation, so that expression of the genes can be modulated. Of interest are regulatory signals which are temperature-sensitive so that by varying the temperature, expression can be repressed or initiated, or are subject to chemical regulation, e.g., metabolite.

Another preferred host is yeast. Yeast provides substantial advantages in that it can also carry out post-translational peptide modifications including glycosylation. A number of recombinant DNA strategies exist which utilize strong promoter sequences and high copy number of plasmids which can be utilized for production of the desired proteins in yeast. Yeast recognizes leader sequences on cloned mammalian gene products, and secretes peptides bearing leader sequences (i.e., pre-peptides).

Any of a series of yeast gene expression systems incorporating promoter and termination elements from the actively expressed genes coding for glycolytic enzymes produced in large quantities when yeast are grown in mediums rich in glucose can be utilized. Known glycolytic genes can also provide very efficient transcription control signals. For example, the promoter and terminator signals of the phosphoglycerate kinase gene can be utilized.

Once the vector or DNA sequence containing the construct(s) has been prepared for expression, the DNA construct(s) may be introduced into an appropriate host. Various techniques may be employed, such as protoplast fusion, calcium phosphate-precipitation, electroporation or other conventional technique. After the fusion, the cells are grown in a selective medium, where untransformed cells are killed, leaving only cells transformed with the DNA construct. Expression of the gene(s) results in assembly to form the hybrid immunoglobulin molecule.

The host cells will for the most part be immortalized cells, particularly myeloma or lymphoma cells. These cells may be grown in an appropriate nutrient medium in culture flasks or injected into a synergetic host, e.g., mouse or rat, or immunodeficient host or host site, e.g., nude mouse or hamster pouch. Particularly, the cells may be introduced into the abdominal cavity for production of ascites fluid and harvesting of the chimeric receptor. Alternatively, the cells may be injected subcutaneously and the antibodies harvested from the blood of the host. The cells may be used in the same manner as the hybridoma cells. See Diamond et al., N. Eng. J. Med. 304:1344 (1981), and Kennatt et al. (eds.), Monoclonal Antibodies: Hybridomas--A New Dimension in Biologic Analysis, Plenum, 1980, which are incorporated herein by reference.

The hybrid immunoglobulin molecule may be isolated and purified in accordance with conventional conditions, such as extraction, precipitation, chromatography, affinity chromatography, electrophoresis, or the like. The preferred method is affinity chromatography with either the amino terminal heptapeptide of the fibrin beta chain (binds to the antifibrin site) or with benzamidine (binds to the plasminogen activator catalytic site) to selectively isolate the hybrid molecule.

The present invention also provides methods for immunotherapy and immunodiagnosis using the hybrid immunoglobulin molecules. In the immunotherapeutic and immunodiagnostic applications, the hybrid immunoglobulin molecule is administered to a patient, which becomes localized at the site of a thrombus through the fibrin-specific binding site of the hybrid molecule. The thrombus is lysed by the enzyme activity of the plasminogen activator portion of the hybrid molecule. As will be appreciated by one of skill in the art, the specificity of the fibrin specific-plasminogen activator hybrid molecule permits selectivity of attachment to and lysis of the thrombus which reduces the risk of serious side effects, such as hemorrhage.

The hybrid immunoglobulin molecules of this invention may also be used in immunodiagnostic applications, including in vivo immunodiagnosis. In this application, the hybrid molecule is detectably labelled using a radionuclide. The radionuclide must be of the type cf decay which is detectable for a given type of instrument. Further, the radionuclide for in vivo diagnosis should have a half-life long enough that it is still detectable at the time of maximum uptake but short enough that after diagnosis unwanted radiation does not remain in the patient. Coupling of the radionuclides to the protein, and thus to the hybrid molecule, is known in the art and is often accomplished either directly or indirectly using an intermediary functional group. Examples of radioisotopes that can be used for in vivo diagnosis are $^{99}$Tc, $^{123}$I, $^{131}$I, $^{111}$In, $^{97}$Ru, $^{67}$Cu, $^{67}$Ga, $^{68}$Ga, $^{72}$As, $^{89}$Zr, and $^{201}$Tl.

Paramagnetic isotopes for purposes of in vivo diagnosis can also be used according to the methods of this invention. Examples of elements that are particularly useful for use in Magnetic Resonance Energy techniques include $^{157}$Gd, $^{55}$Mn, $^{162}$Dy, $^{52}$Cr, and $^{56}$Fe.

The hybrid immunoglobulin molecule can further comprise a pharmaceutical composition, with a pharmaceutically acceptable carrier. These carriers are well known in the art and can include aqueous or solvent emulsions or suspensions including saline and buffered media. The pharmaceutical art, for example, as described in Remington's Pharmaceutical Sciences (16th Edition, 1980).

The dose ranges for administration of the hybrid immunoglobin molecule are those that are large enough to detect the presence of thrombi. The dosage should not be so large as to cause adverse side effects, such as hypersensitivity reactions such as rashes or anaphylactic shock. Generally, the dosage will vary with the age, condition, sex, and extent of disease in the patient. Counterindications can include hypersensitivity and other variables and can be adjusted by the individual physician. Dosage can range from 0.01 mg/kg to 500 mg/kg of body weight, preferably 0.01 mg/kg to 200 mg/kg. The hybrid immunoglobulin molecule(s) can be administered parentally by injection or by gradual perfusion over time. They can also be administered intravenously, intraperitoneally, intramuscularly, or subcutaneously.

The invention will now be described by way of example with reference to the accompanying drawings in which:

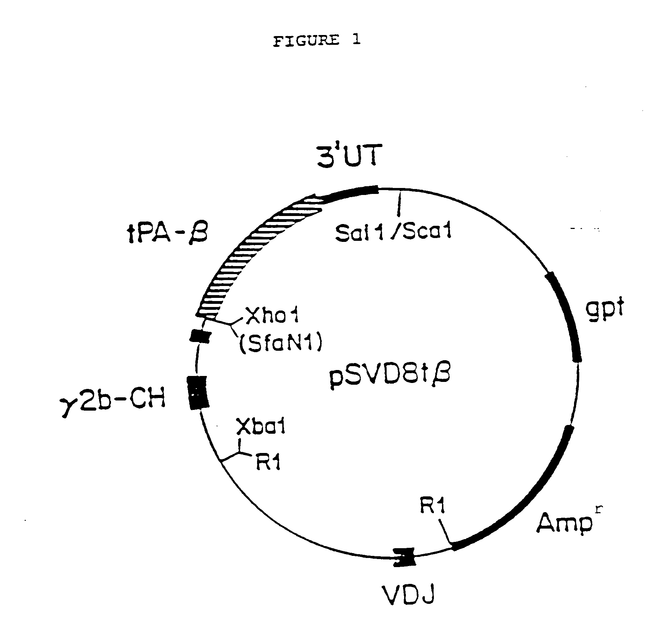

Figure 1 is a map of expression plasmid pSVD8tβ, which codes for the heavy chain-t-PA fusion protein. Coding sequences are indicated by labels outside the circle; restriction sites used in construction are indicated inside the circle. Abbreviations: VDJ, productive 59D8 heavy chain rearrangement; gamma2b-CH, genomic sequence of the murine 2b heavy chain constant region; t-PA-β, cDNA sequence coding for the human t-PA chain; 3'-UT, 3' untranslated sequence of human t-PA cDNA; Amp$^r$, pBR322 ampicillin resistance gene; gpt, *E. coli* guanine phosphoribosyl transferase gene driven by SV40 promoter; RI, Eco R1;

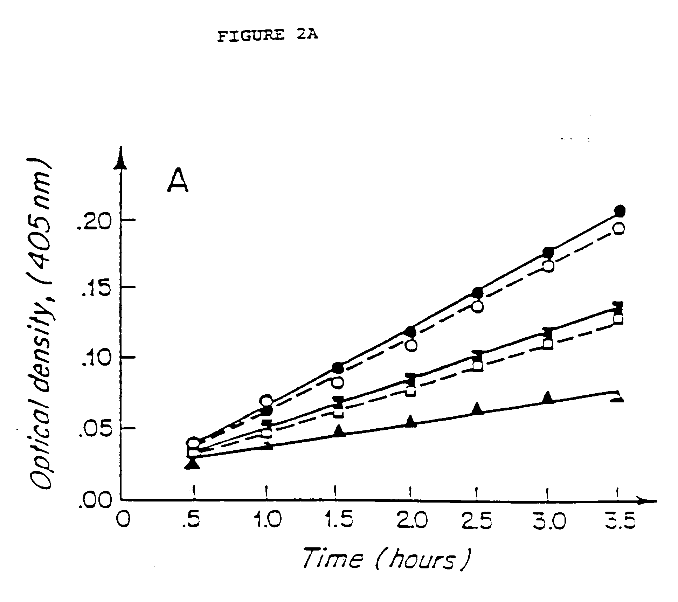

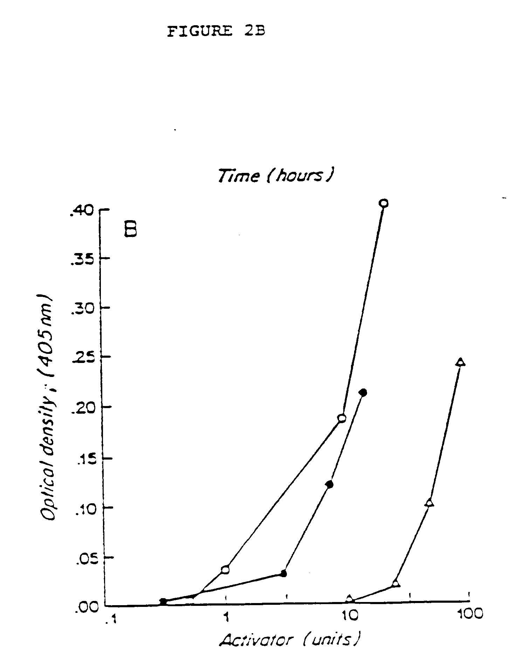

Figures 2A and 2B are graphs of optical density against time or activator units respectively for a chromogenic substrate assay comparing the catalytic activity of the recombinant protein with that of the melanoma t-PA. In Figure 2A, as shown by the dashed lined, the S-2288 assay was performed with 105 ng (open circles) or 70 ng (open boxes) of recombinant protein. Solid lines represent the catalytic activity of 50 ng (filled circles), 40 ng (filled boxes), or 30 ng (filled triangles) of melanoma t-PA used as standards. Relative molar activity of the recombinant protein was determined by comparison with t-PA standards with similar rates of catalysis. In Figure 2B the S-2251 assay was conducted with varying activities of melanoma t-PA standard (open circles), recombinant protein (filled circles), and bovine trypsin (open triangles). Units of activity of each protein were determined in the S-2288 assay.

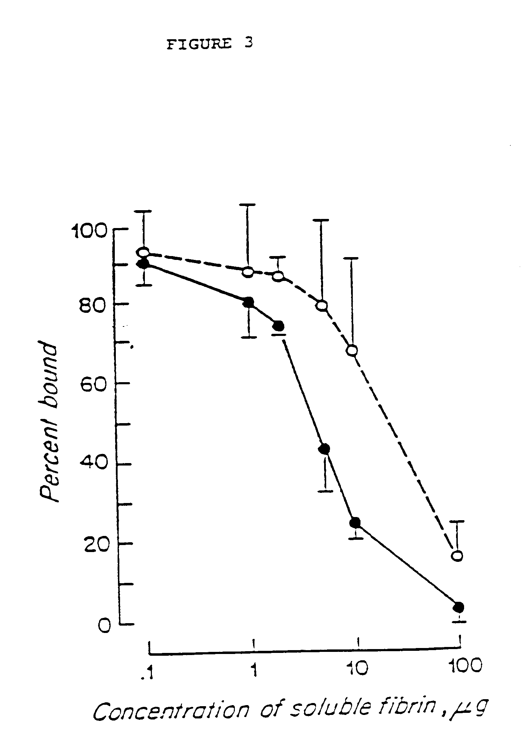

Figure 3 is a graph of binding against fibrin concentration in a comparison of the binding behaviour of 59D8 antifibrin antibody and recombinant protein. Curves represent the inhibition of antibody to solid-phase fibrin monomer by competition with various concentrations of soluble fibrin monomer. Recombinant antibody (dashed lines) requires a slightly higher concentration of soluble fibrin for 50% inhibition than does the antibody (solid lines) and thus binds fibrin somewhat less avidly. This difference is less than 10-fold.

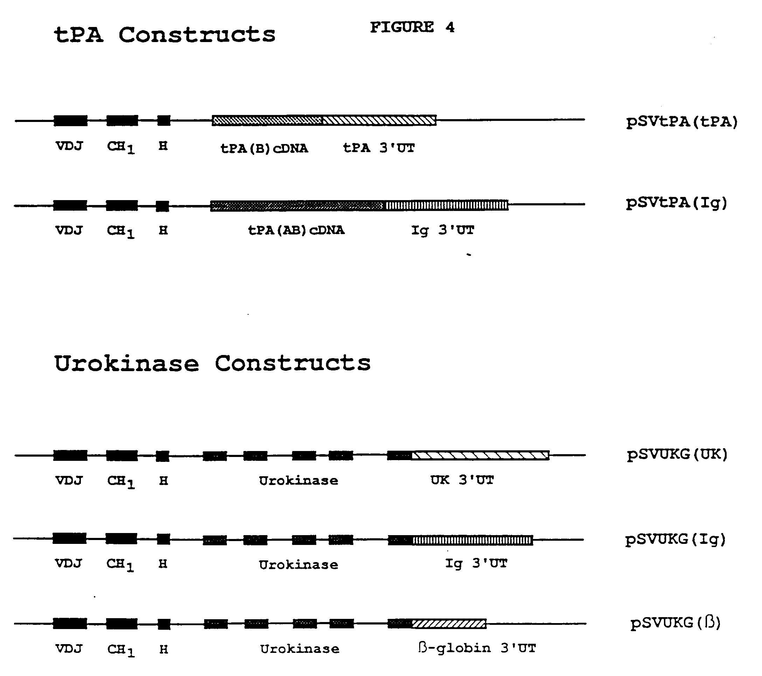

Figure 4. Genes transfected into 59D8 L2LV cells. pSVtPA(tPA) contains a genomic variable (VDJ) region from fibrin-specific monoclonal antibody 59D8, cloned genomic constant region [CH1,H, and (hinge)] of the mouse gamma 2b antibody, and cDNA sequence coding for the B-chain of tPA (amino acids 275 to 527) and the tPA 3'untranslated region. In pSVtPA(Ig), the tPA 3' UT domain has been replaced by 3' untranslated region from the gamma 2b Ig gene. In addition, the protein encoding sequence has been expanded to include both the A and B chains of tPA (amino acids 1 to 527).

pSVUKG(UK) contains the 59D8 VDJ exon, gamma 2b constant regions, the coding regions from a genomic clone of single-chain urokinase (scuPA) containing axons VII through XI, and a 3' UT sequence from the urokinase gene. Two modified chimeric genes were made by substituting either the 3' untranslated region of β-globin [pSVUKG(β)] or the 3' UT of the mouse γ2b Ig gene [pSVUKG(Ig)].

All genes were assembled in the pSV2gpt vector to form their respective expression plasmids. The pSV2gpt vector contained an SV-40 promoter-driven <u>Escherichia coli</u> xanthine guanine phosphoribosyl transferase (gpt) gene, serving as the selective marker in transfected hybridoma cells and a partial pBR322 sequence for plasmid replication and clone selection in <u>E. coli</u>.

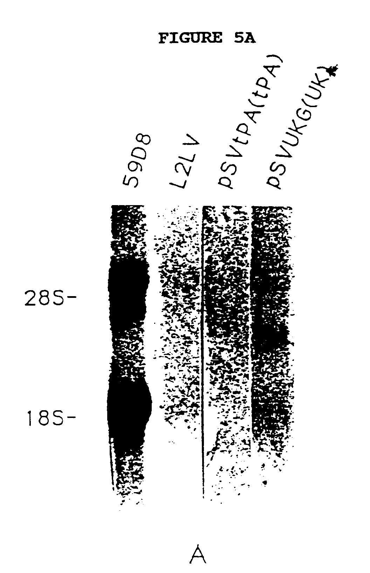

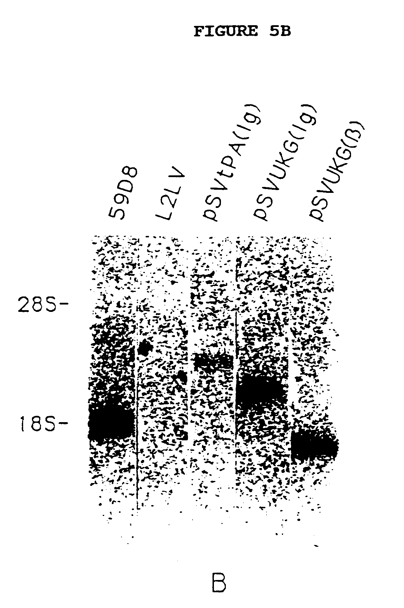

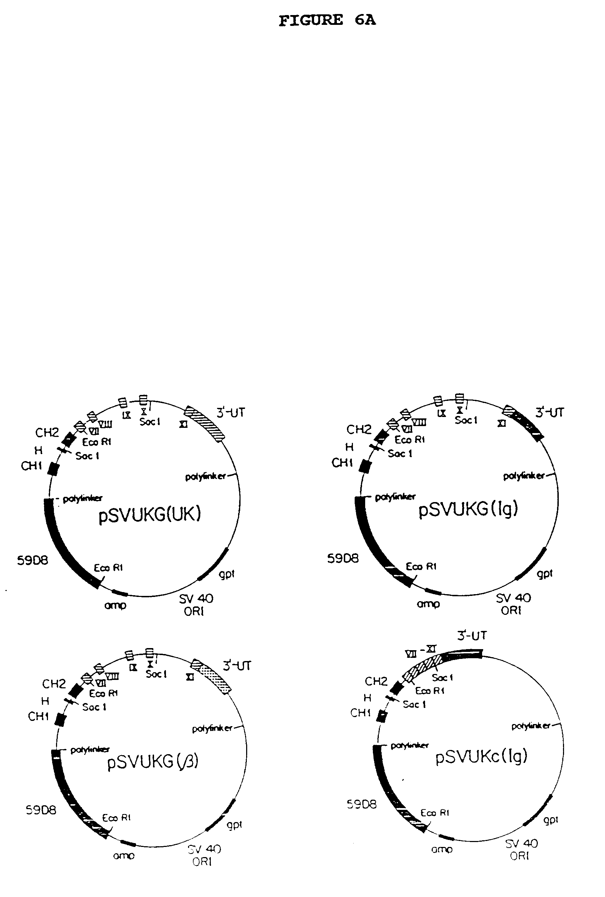

Figures 5A and 5B show RNA transfer blot analysis. Equal amounts of total cellular RNA run in each lane from each of the five transfectomas, parental 59D8 cells, and heavy chain loss variant cells (L2LV). Panel A compares pSVtPA(tPA) and pSVUKG(UK) to 59D8 cells. Panel B compares pSVtPA(Ig), pSVUKG(Ig), and pSVUKG(β) to 59D8 cells. L2LV cells serve as a negative control. Figure 6A gives the maps for expression plasmids for r-scuPA(32)-59D8. (A) pSVUKG(UK) contains a genomic heavy-chain variable region from fibrin-specific monoclonal antibody 59D8, cloned genomic constant region of the mouse γ2b [CH1, H (hinge) and CH2], and the coding region from a genomic clone of scuPA(32) (containing axons VII through XI). In pSVUKG(UK) the 3' UT region is that of scuPA, beginning at Leu$^{144}$. Also contained in this plasmid are an SV-40 promoter-driver <u>Escherichia coli</u> xanthine guanine phosphoribosyl transferase (gpt) gene, serving as the selective marker in transfected hybridoma cells, and a partial pBR322 sequence for plasmid replication and clone selection in <u>E. coli</u>. Three modified plasmids were made by substituting either the 3' UT region of β globin (Lawn, R., <u>et al.</u>, <u>Cell 21</u>:647 (1980)) [pSVUKG(β)], or the 3'UT region of mouse immunoglobulin (from antibody 59D8) [pSVUKG(Ig), and pSVUKc(Ig)]. pSVUKc(Ig) also differed in that the genomic DNA encoding scuPA(32) was replaced by cDNA encoding the same region (axons VII through XI).

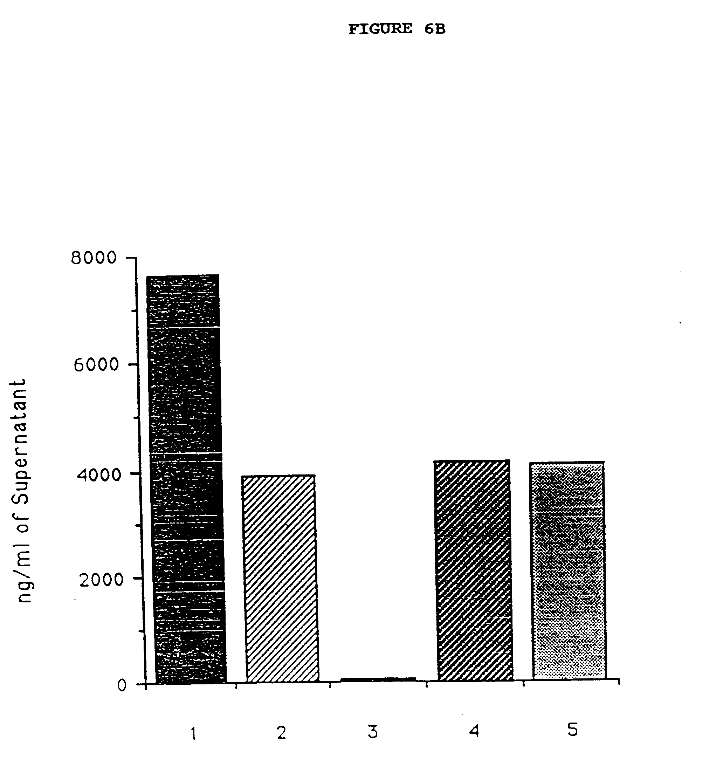

Figure 6B gives protein expression levels.

Mouse L2LV cells [stable, subcloned heavy-chain loss variant cells derived from hybridoma cells producing native 59D8 (Schnee, J.M., <u>et al.</u>, <u>Proc. Natl. Acad. Sci. USA 84</u>:6904 (1987); Love, T.W., <u>et al.</u>, In <u>Methods in Enzymology</u>, Langone, J.J. (ed.), Academic Press, New York, pp. 515-527 (1989))] were harvested at log phase and transfected with linearized plasmid (20 μg/1.0 ml of cell suspension) by electroporation (200 volts and 960 μFD). After selection for stable clones and subclones, cells were grown to confluence in 100-mm petri dishes. The supernatants were harvested at equal cell densities. The ng/ml values for supernatants are based on the presence of mouse immunoglobulin measured by reference to a standard curve (as described in Figure 3B). Results represent the means of duplicate determinations. Samples are (1) supernatant from 59D8 cells, (2) supernatant from γ2b cells (heavy-chain loss variant cells that had been transfected with an expression plasmid encoding only the heavy chain of antibody 59D8), (3) supernatant from pSVUKG(UK), (4) supernatant from pSVUKG(β), and (5) supernatant from pSVUKG(Ig).

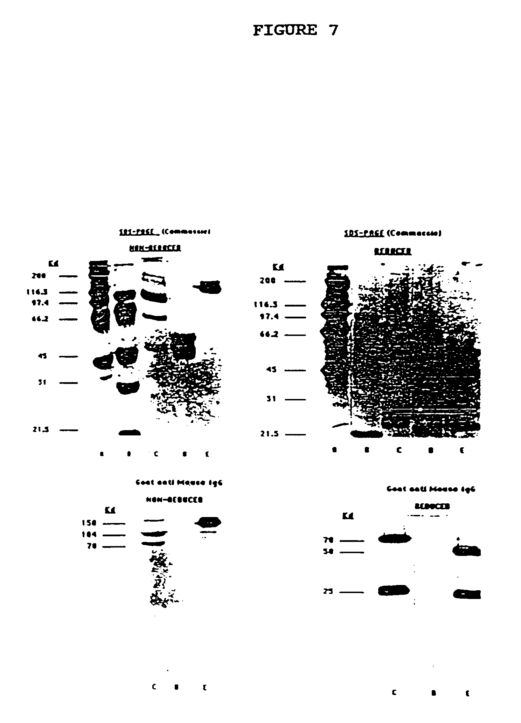

Figure 7 shows an SDS-polyacrylamide gel electrophoresis, performed according to the method of Laemmli (Laemmli, U.K., Nature 227:680 (1970)) as described (Runge, M.S., et al., Biochemistry 27:1153 (1988)). Proteins were either visualized with Coomassie Brilliant Blue R [(A) nonreduced, (B) reduced] or transferred by electrophoresis (Bode, C., et al., Circulation (in press)) to a nitrocellulose filter. These filters were blocked with a 1% BSA solution and then probed with phosphatase-conjugated goat-antimouse IgG (ELISAmate kit, Kirkegaard and Perry Laboratories, Gaithersburg, MD) [(C) nonreduced, (D) reduced]. Lanes A and B, molecular weight standards (in kDa); 200, myson; 116.3, β galactosidase; 97.4, phosphorylase B; 66.3, bovine serum albumin; 45, ovalbumin; 31, carbonic anhydrase; and 21, soybean trypsin inhibitor. Lane C, purified 4-scu-PA(32)-59D8. Lane D, scuPA (purified scuPA from Sandoz contains human serum albumin, as a stabilizer, present at approximately 66 kDa]. Lane E, affinity-purified antibody 59D8. Molecular weight calculations were made from unknown bands on the basis of relative mobility (in both reduced and nonreduced gels) in comparison with the known standards.

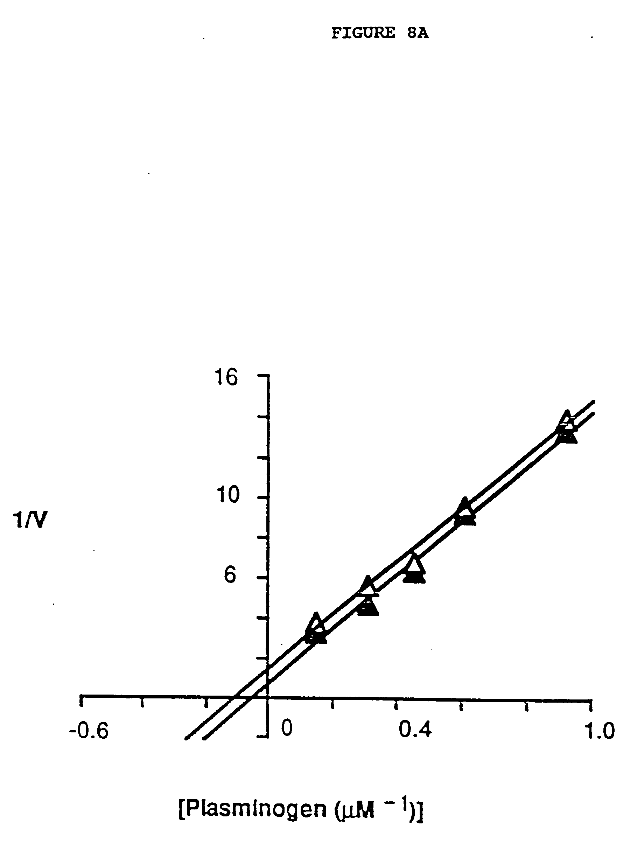

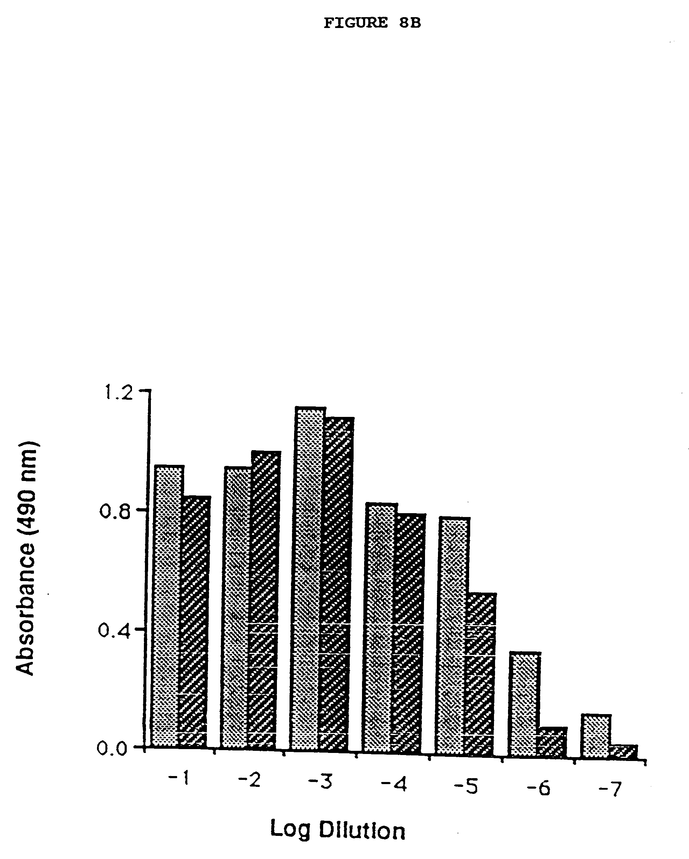

Figures 8A and 8B are graphs illustrating the kinetic and fibrin binding properties of r-scuPA(32)-59D8. (A) Initial rates of reaction for two-chain urokinase (Abbott Laboratrries, Abbott Park, IL) and the two-chain form of r-scuPA(32)-59D8 were measured by changes in absorbance at 405 nm resulting from the cleavage by plasmin of S-2251 (Helena Laboratories, Beaumont, TX); the protocol was adapted from that of Dewerchin et al. (Dewerchin, M., et al., Eur. J. Biochem 185:141 (1989)). Points represent the means of duplicate determination, and their general pattern was reproduced in several different experiments. Data obtained for urokinase and the two-chain form of r-scuPA(32)-59D8 did not significantly differ. Figure 8B shows fibrin binding by native 59D8 (stippled bars) and r-scuPA(32)-59D8 (hatched bars). ELISA plates (96-well; Falcon) were coated with fibrin monomer (5 μg/ml). After blocking, samples of either 59D8 or r-scuPA(32)-59D8 were incubated on the plates [serial dilutions of 59D8 or r-scuPA(32)-59D8 ranged between $1.7 \times 10^{-3}$ and $1.7 \times 10^{-6}$ mg (of antibody)/ml] for one hour to allow binding. The wells were washed extensively with Tris-saline (pH 8, with 0.05% Tween-20) and blocked again with BSA. The wells were then probed with a Fab′$_2$ preparation of polyclonal rabbit anti-mouse IgG antibodies that had been labelled with biotin. Then the plates were treated with ELISA-amplification reagents (Bethesda Research Laboratories, Bethesda, MD) to obtain a colour reaction. The means of duplicate determinations are shown.

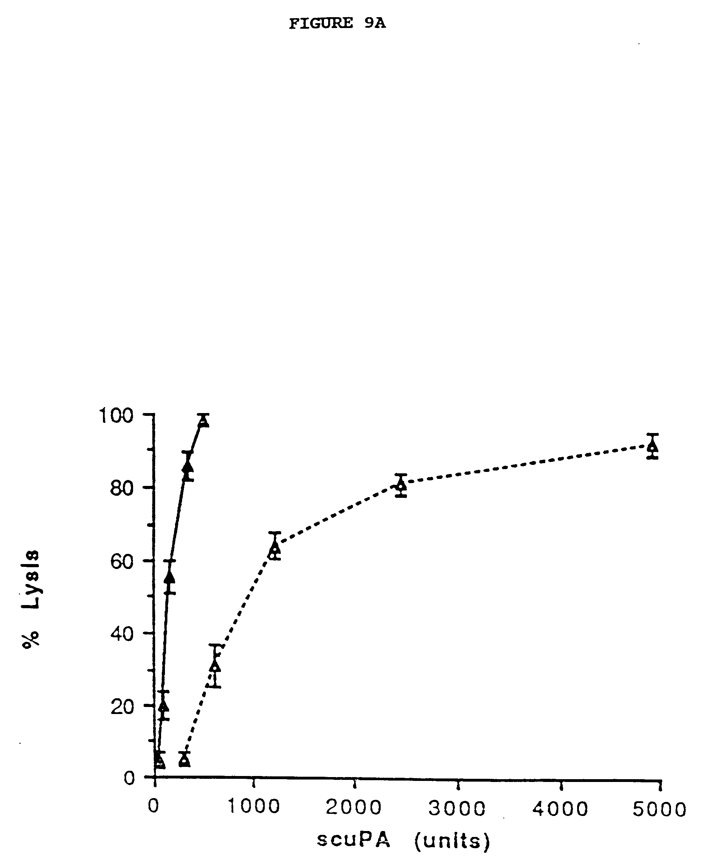

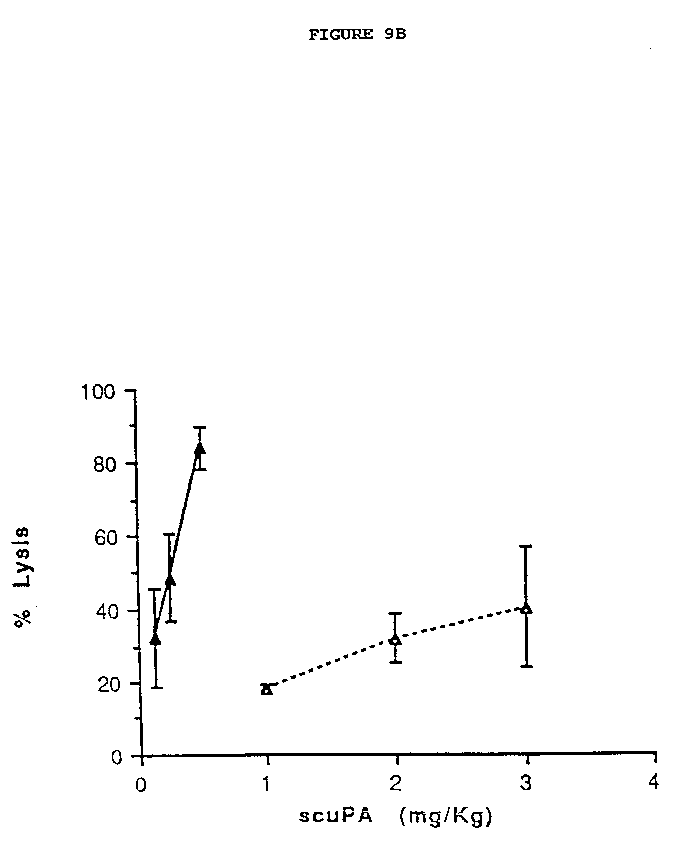

Figures 9A and 9B are graphs of percentage lysis against amount of scuPA. Figure 9A is a human plasma clot lysis assay, performed as described (Runge, M.S., et al., Biochemistry 27:1153 (1988)) on the basis of Bode, C., et al, Circulation (in press). Plasminogen activator concentrations were based both on protein concentration and S-2444 activity (see above). Points represent clot lysis at 2 hours. "Fold increase in potency" was calculated by fitting the percent lysis curves in the plasma clot and rabbit jugular vein assays to anti-logic functions of two parameters (estimated) that have been shown to fit curves of percent lysis versus does of plasminogen activator (Bode, C., et al, J. Mol. Cell. Cardiol. 19:335 (1987)). Figure 9B shows thrombolysis in vivo. The rabbit jugular vein model of Collen et al. (Collen, S., et al., J. Clin. Invest. 71:368 (1983)) was modified as described (Runge, M.S., et al., Proc. Natl. Acad. Sci. USA 84:7659(1987); Collen, D., et al., Fibrinolysis 3:197 (1989)). Plasminogen activators (or saline) were administered by infusion of a bolus (consisting of 20% of the total dose) over one minute, along with a heparin bolus (300 units/kg) over one minute, followed by continuous infusion over the next sixty minutes of the remainder of the plasminogen activator dose and continuous infusing of heparin over the next 180 minutes (60 units/kg per hour). The animal was killed after this three-hour treatment and the amount of thrombolysis was measured by gamma counting of the remaining vein segment. Data represent the means of values from between 3 and 8 animals at each point. The 20-fold increase in potency for r-scuPA(32)-59D8 was derived as described in A.

The invention will be further described by reference to the specific examples which are included herein for purposes of illustration only, and are not intended to be limiting unless otherwise specified.

EXAMPLE 1

Materials.

N-succinimidyl 3-(2-pyridyldithio) propionate (SPDP) and 2-iminothiolane were obtained from Pierce, and Sepharose 4B-CL was obtained from Pharmacia. The $^{125}$I fibrinogen (IBRIN) came from Amersham. Plasma was purchased from the local blood bank. A chromogenic substrate for proteases H-D-isoleucyl-L-prolyl-L-arginine-p-nitroanalide dihydrochloride (S-2288) was obtained from Helena Labs. All other chemicals came from either Sigma or Fisher.

Fibrin-specific antibody 64C5 has been described in Hui et al., supra, and in commonly owned and co-pending United States Patent Application, Serial No. 824,228, incorporated herein by reference. Fibrin-specific antibody 59D8 has been described in commonly owned and co-pending United States Patent Application, Serial No. 851,514. incorporated herein by reference.

Electrophoresis and autoradiography.

SDS-PAGE was performed according to the method of Laemmli, Nature (London) 277:681 (1970). Proteins were visualized using either Coomassie Brilliant Blue R or, where radiolabeled, by autoradiography for 24-72 hours at -70°C.

Cloning the urokinase gene.

A complementary DNA clone (PHUK8) containing the catalytic B chain coding sequence was utilized (Verde et al., Proc. Nat'l. Acad. Sci. USA 81:4727 (1984)). This clone has been grown and isolated in quantity in pBR322. A segment of this DNA clone is used in constructs with antifibrin antibody genes, and it is used to isolate the genomic DNA sequence.

Cloning the tPA gene.

A full length complementary DNA clone of the human tPA gene (PPA34'F) was utilized. The sequence coding for the catalytic B chain of tPA has been extracted from the clone and a unique short segment of synthetic DNA (adapter) coding for two common restriction enzyme cleavage sites placed at the 5' end of this sequence. This adapter allows the gene to be conveniently joined "in-frame" to other segments of DNA. This tailored clone is used in constructs with antifibrin antibody genes. It is also used to isolate the genomic sequence coding for the tPA β chain, and the genomic sequence subsequently used in constructs with the 59D8 genes.

Cloning the variable light and heavy chain genes of the fibrin-specific antibody 64C5.

Using a redundant oligonucleotide 17-mer, which was synthesized to correspond to the amino terminus for the light-chain of 64C5 and an available kappa/J probe, the productive 64C5 light chain rearrangement was cloned from a subgenomic library constructed in pBR322. The entire light chain was reconstructed by ligating the cloned rearranged fragment into a pR322 plasmid that contains a 5.8 Kb EcoR1/BamH1 fragment which encodes the mouse kappa constant region and joining segments (Max, et al., J. Biol. Chem. 256:5116-20 (1981)). The VJ rearrangement was placed 5' of the constant region in the correct orientation. Genomic DNA derived from the 64C5 hybridoma was digested with EcoR1 restriction enzyme, size fractionated on a preparative agarose gel, and subsequently ligated into a lambda cloning vector to produce a subgenomic library. Using a probe from the heavy chain joining region. a rearranged fragment was cloned. It was determined to be the heavy chain rearrangement used in the 64C5 hybridoma by hybridization to an oligonucleotide based on the amino terminal sequence of the antibody.

Cloning the variable heavy chain gene of the fibrin-specific antibody 59D8.

Genomic DNA from the 59D8 hybridoma was size fractionated, ligated into a lambda cloning vector and screened with the heavy chain joining region probe noted above. Both heavy chain rearrangements were cloned and the productive one was selected by hybridization to an oligonucleotide 20-mer based on RNA sequence of the 59D8 heavy chain.

Fibrin-specific antibody/plasminogen activator genetic constructs.

The cloned restriction fragment, containing variable and joining region as well as enhancer sequences of the 59D8 or 64C5 gene, is inserted in correct orientation into a plasmid 5' of the mouse gamma 2B heavy chain constant region sequence. This plasmid containing the constant region sequence (PSV GPT/gamma 2B) also contains the ampicillin resistance gene from pBR322, and the guanine phosphoribosyl transferase (GPT) gene under control of the SV40 viral promoter. This construct is propagated in E. coli MC1061 via the ampicillin resistance gene, and expression of the GPT gene in eukaryotes could be selected for in the presence of xanthine, hypoxanthine, and mycophenolic acid. The bulk of the sequence coding for the carboxy terminus of the heavy chain constant region was subsequently removed. It has been replaced with a complementary DNA fragment which codes for the catalytic carboxy "B" chain or urokinase or tissue plasminogen activator. The third exon from either one of the heavy chain constant region genes is joined "in frame" to one of the plasminogen activator genes such that the usual amino acid sequence will be produced, and a composite protein will result. This final construct is transfected via electroporation into the appropriate 59D8 or 64C5 hybridoma variant which has stopped producing the usual heavy chain. These transfectants produce an antibody molecule with fibrin specificity, with a plasminogen activator moiety at the tail end of the truncated heavy chain.

## Purification of urokinase-64C5 hybrid immunoglobulin molecules.

The hybrid molecule is isolated utilizing successive affinity chromatography on benzamidine-Sepharose and beta-peptide-Sepharose in order to obtain hybrid molecules that contain both an antibody combining site and urokinase or tPA sequence. The affinity column is constructed by coupling a synthetic amino terminal beta chain fibrin peptide (Gly-His-Arg-Pro-Leu-Asp-Lys-Cys (beta peptide)) Hui, et al., Science 222:1129 (1983) to maleimidokenzoyl lysine-Sepharose C1-4B (Kitagawa, et al., J. Biochem. (Japan) 79:233 (1976). The eluate of this column (0.2 M glycine HCl, pH 2.8) is recovered and analyzed to determine fibrin binding properties and fibrinolytic activity.

## Purification of tPA-59D8 hybrid immunoglobulin molecules

Hybrid immunoglobulin molecules are purified from the host cells by sequential affinity chromatography in two steps. The molecules are first applied to Sepharose CL-4B containing immobilized benzamidine: tPA-antibody hybrid molecules are retained and later eluted with 0.1 M acetate, 0.4 M NaCl (pH 4.0). After neutralization, the eluate is then applied to the peptide-Sepharose column. The eluate from the beta peptide column (0.2 M glycine, pH 2.8), is dialyzed against NaPi buffer including 1.0 M arginine and 0.1% Tween 80 and was stored in this buffer at 4 degrees.

## Characterization of hybrid immunoglobulin molecules.

The hybrid molecules are subjected to SDS-PAGE under both reducing and nonreducing conditions. The gels are either stained with Coomassie Blue or subjected to autoradiography (if tPA or UK is labeled with $^{125}$I before coupling).

## Chromogenic substrate assay for peptidase activity.

To assess the functional properties of the hybrid molecule, its peptidolytic properties are first examined with respect to a nonselective substrate, H-D-isoleucyl-L-prolyl-L-arginine-p-nitroanalide dihydrochloride (S-2288). The S-2288 assay is performed with a total volume of 1.0 ml in 0.05 M Tris-HCl, 0.10 M NaCl (pH 8.5) with a substrate concentration of $3 \times 10^4$ M. Absorbance at 405 nm is measured every 10 seconds at 20°C.

## Fibrinogen assays.

The fibrinogen content of samples of citrated human plasma or citrated rabbit plasma was determined by two methods. Clottable fibrinogen was measured by the method of Clauss, Acta Chir. Scand. 90:419 (1957), and total fibrinogen is determined by sodium sulfite precipitation.

## Plasma clot assay.

The method of Lijnen et al., Thromb. Haemostas. 52:308 (1984) is used with the following modifications. Human fresh-frozen plasma obtained from donors is pooled, aliquoted, and refrozen. Immediately before each experiment, the activities of tPA, UK and their hybrid immunoglobulin molecules are calibrated using the S-2288 assay (i.e., the peptidase activities of the native plasminogen activators and their hybrid molecules are determined and appropriate dilutions made so that the peptidase activity [in units/ml] is identical for each sample). Plasma clots are made by adding each of the following to fresh-frozen plasma: thrombin 8 NIH units/ml; 0.5 M $CaCl_2$, 100 ul/ml; and $^{125}$I-labeled human fibrinogen (IBRIN), 40,000 cpm/ml. The solution is immediately drawn into Silastic tubing (I.D. = 4 mm), and incubated at 37°C for 30 min. Silastic tubing containing clotted fresh-frozen plasma is cut into 1.5-cm sections, yielding clots of 0.2 ml. These are then washed in 0.15 M NaCl before use. Each clot is placed in a plastic tube, counted, and suspended in 1 ml fresh-frozen plasma (from the same pool). Experiments are initiated by the addition of a plasminogen activator (or hybrid molecule of plasminogen activator and antibody). At 30 minute intervals, an aliquot of the fresh-frozen plasma is removed from each tube for counting. Samples are saved at the end of the experiment for determination of fibrinogen levels.

In vivo thrombolysis.

The rabbit jugular vein model of Collen et al., J. Clin. Invest. 71:368 (1983), is used. After sedation of the rabbit with acetopromazine and ketamine, a paramedial incision is made from the right mandible to above the right clavicle. The external jugular vein is isolated by dissection, and branches are ligated and separated. A segment of woolen thread is introduced to anchor the cot. After bleeding ceases, vascular clamps are placed so as to isolate this segment of the external jugular vein, and the components of the clot are introduced into the isolated vein segment. These components consist of approximately 500,000 cpr of $^{125}$I-labeled human fibrinogen (each sample is counted before use), 100 ul of packed human red blood cells, 100 ul of human fresh-frozen plasma, 10 ul of 0.5 M $CaCl_2$ and 10 ul of bovine thrombin (8 NIH units). After 30 minutes, the vascular clamps are removed and blood flow is restored. A sample of blood is taken immediately after the clamps are released to determine radioactivity not incorporated into the thrombus. Measured amounts of plasminogen activator are diluted to a volume of 25 ml, and are delivered via the marginal vein of the contralateral ear over 4 hours by infusion pump. Lost counts are determined by counting syringes, gauze sponges and tubing. Six hours after initiation of the infusion, the entire vein segment is isolated, removed and counted. Percent lysis is determined as the ratio of the counts remaining at the termination of an experiment over the net counts at the beginning.

Fibrinolytic assay.

The quantitative fibrinolytic assay links fibrin monomer to Sepharose. Fibrinogen is purified of plasmin contaminates by passage over lysine-Sepharose and then mixed with trace-labelled $^{125}$I-fibrinogen. It is then coupled to cyanogen bromide activated Sepharose C1-4B. The immobilized fibrinogen is converted to fibrin by addition of human thrombin in the presence of 100 mM $CaCl_2$.

To assess their relative fibrinolytic activity, increasing amounts of $^{125}$I-UK-64C5 hybrid molecule and urokinase (or $^{125}$I-tPA-59D8 hybrid molecule and tPA) are incubated with $^{125}$I-fibrin-Sepharose for 4 hours. Thereafter, the resin is incubated with purified plasminogen. After intervals of 2.5 and 15 hours, the mixture is centrifuged and the radioactivity of the supernatant is determined. This procedure is repeated with the control conjugate, ($^{125}$ I-UR)-SS-(3H3).

EXAMPLE 2

MATERIALS AND METHODS

Cloning of 59D8 Heavy Chain Gene.

High molecular weight genomic DNA was made from the 59D8 hybridoma cells as previously described in Quertermous et al., J. Immunol. 128:2687-2690 (1987). To identify rearranged heavy chain immunoglobulin genes specific for the 59D8 hybridoma line, Southern blot analysis was performed as previously described with Eco R1-digested genomic DNA and a 1.7-kilobase (kb) Eco R1/Pst1 genomic joining region probe (Southern, E.M., J. Mol: Biol. 98:503-517; Sakano et al., Nature 286:676-683 (1980). Two rearrangements were identified that were not found in either of the cells originally fused to produce the 59D8 hybridoma (SP2/0 and Balb/c). Subsequently, one milligram of genomic DNA was digested with Eco R1 and size-fractionated on a preparative agarose gel. Southern, E., in Methods in Enzymology et. Wu, R. (Academic Press, NY), Vol. 68, pp. 152-176. Fractions containing each of the two rearranged fragments were identified by hybridization to the joining region probe. These fractions were concentrated an ligated into λgt10. The two subgenomic libraries thus constructed were screened with the joining region probe and several potential clones were isolated from each library. (Maniatis et al., Molecular Cloning, 1982 (Cold spring Harbor, NY). Selection of the clone containing the rearranged fragment coding for the 59D8 antigen combining site was accomplished by hybridization to a 20-basepair oligonucleotide that had been constructed on the basis of the sequence of the 59D8 heavy chain mRNA. RNA isolation and sequencing, $^{32}$P labeling of the oligonucleotide with T4 polynucleotide kinase, and hybridization were carried out according to previously described techniques. (Maniatis et al., Molecular Cloning, supra; Clarke et al., J. Exp. Med. 161:687-704 (1985); Suggs et al., Proc. Nat'l Acad. Sci. USA 78:6613-6617 (1981).

Expression Vector Construction.

The t-PA sequence was derived from a cDNA clone (pPA34'F) that had been constructed from HeLa cell mRNA. Fisher et al., J. Biol. Chem. 260:11223-11230 (1985). DNA encoding the β chain SacI site to the Eco R1 site of pBR322 was isolated and ligated into pGEM3. Next, a contiguous 5′ fragment was isolated by digestion with SfaN1 and Sac1. A synthetic oligonucleotide, containing a Bam H1 end, an XhoI site, and two bases reconstituting a codon for glycine, was added to this second fragment's 5′ end. The modified fragment was then ligated into a plasmid already containing the 3′ fragment, thus reconstituting the chain sequence. The chain was excised with Xho1 and Sca1 - the Sca1 site being contributed by the pBR322 sequence.

The final construct was assembled in the pSV2gpt vector that had been modified by the insertion of a polylinker containing a 6-kb Xba1 restriction fragment encoding the murine $\lambda$2b heavy chain constant region. Mulligan et al., Proc. Nat'l Acad. Sci. USA 78:2072-2076 (1981); Tucker et al., Science 206:1303-1306 (1979). The productive 59D8 heavy chain rearranged gene that had been cloned on a 2.6-kb Eco R1 fragment was inserted in the correct orientation into an Eco R1 site in the polylinker 5′ of the $\gamma$2b constant region. The constant region sequence between the unique Xho1 site in CH2 and a Sal1 site in the polylinker was excised, the Sal1 site was blunted and the t-PA chain was ligated into place. Nucleotide sequence analysis confirmed that the junction between the heavy chain and t-PA segments was in-frame. Sanger et al., Proc. Nat'l Acad. Sci. USA 74:5463-5467 (1977).

Monoclonal Antibodies and Selection of Loss Variants.

Fibrin-specific monoclonal antibody 59D8 was raised by immunization with a synthetic heptapeptide based on the amino terminal sequence of the fibrin chain, as previously described in Hui et al., Science 222:1129-1132 (1983). Hybridoma cells and loss variants were maintained in complete medium: DMEM with 4.5 mg/ml glucose, 12 percent fetal calf serum (FCS), 50 g/ml gentamicin sulfate, and 0.6 mg/ml L-glutamine. For selection of heavy chain loss variants, cells were grown in soft agarose. Five milliliters of complete medium plus 0.2 percent agarose and an additional 8 percent FC5 was added to tissue culture dishes (60 mm) and allowed to solidify at room temperature for 3 to 5 min. Cells (1 to 2 x $10^3$) to be selected for chain loss were layered over the agarose. The plates were incubated at 37°C in 6 percent $CO_2$ until clusters of cells were formed (2 to 4 days). To detect heavy chain loss variants, cell clusters were overlayed with an antiserum solution 1.0 ml) containing complete medium with 0.2 percent agarose and 5 to 10 percent rabbit or goat anti-mouse heavy chain. Cell clusters secreting heavy chain developed a precipitin halo. Clusters that did not have a precipitin halo were picked from soft agarose by capillary pipet and sub-sequently delivered into 96-well plates containing complete medium with 8 percent additional FC5. Individual subclones were assayed by enzyme-linked immunoabsorbent assay (ELISA) or by Western blotting for the presence of heavy and light chain.

Transfection and Selection.

The construct pD85Vt$\beta$ was transfected into loss variant cells by electroporatin using an Isco power supply as described in Potter et al., Proc. Nat'l Acad. Sci. USA 81:7161-7165 (1984). Optimal transfection conditions were a 2000-volt discharge into 0.8 ml of phosphate buffered saline. Transformants were selected by growth in mycophenolic acid, xanthine and hypoxanthine. Confirmation of transfection and expression was obtained by Northern blot analysis using a 2-kb cDNA probe coding for the 3′ portion of the human t-PA$\beta$ chain. Maniatis et al., Molecular Cloning supra. Transfected cell lines were subcloned according to standard techniques.

Protein purification.

Protein was purified from cell supernatants and from ascites by sequential double affinity chromatography on two columns. One column was constructed by linking the synthetic peptide used for the generation of 59D8 to Sepharose. The other consisted of an anti-human t-PA monoclonal antibody linked to Sepharose. We had used a third column, composed of benzamidine linked to Sepharose, in our initial purification attempts. However, even though benzamidine binds well to the active site of t-PA and benzamidine-Sepharose can be used to purify the intact molecule, the column did not retain the recombinant protein.

Purification of the recombinant protein was monitored by two solid-phase immunoassays. To detect antifibrin antibody activity, 96-well microtiter plates were coated with fibrin monomer and blocked with 10 percent horse serum. They were then incubated with samples, and washed and probed with $^{125}$I-labeled goat anti-mouse Fab. The second assay was designed to detect t-PA antigen associated with antifibrin antibody activity. In this assay, the fibrin-monomer-coated plates were incubated with culture supernatant or ascites and probed with $^{125}$I-labeled anti-human t-PA. Because the chain of t-PA possesses no fibrin binding activity, only recombinant protein containing both functional domains is detected.

Western blot analysis.

Western blots were made from both reduced and nonreduced samples separated on $NaDodSO_4$ polyacrylamide gels using established techniques. Burnette, W.N., Anal. Biochem. 112:195-203 (1981). Either goat anti-mouse Fab or a monoclonal anti-human t-PA antibody labeled with $^{125}$I was used as a probe.

Antigen binding assay.

The original antibody (59D8) and the recombinant molecule were first assayed for the presence of fibrin-binding Fab antigen. This was accomplished with the solid-phase immunoassay described above using $^{125}$I-labeled goat anti-mouse Fab as a probe. Titration curves were generated for 59D8 and the recombinant protein by varying their concentrations in the assay. That concentration which would yield the same amount of bound $^{125}$I-labeled antibody was then selected from the linear part of each curve. At this concentration of either 59D8 or fusion protein, a competition assay was performed in wells that had been coated with fibrin and filled with various amounts of soluble fibrin. Protein that bound to the soluble rather than insoluble fibrin was removed by washing before application of the labeled antibody.

Assays of enzymatic function.

To compare the enzymatic function of the recombinant protein with that of native t-PA, its peptidolytic properties were first examined in an assay which measures cleavage of the nonselective substrate S-2288 (Helena Labs, Beaumont, TX). The assay was carried out in a 50 $\mu$l volume of buffer (0.15 M Tris, 015 M NaCl) with a 1 millimolar final concentration of chromogenic substrate. Various concentrations of recombinant protein or t-PA purified from the Bowes melanoma cell line (Bio Response, Hayward, CA) were added and the absorbance at 405 nm was measured at a series of time points.

To determine whether the recombinant protein was capable of activating plasminogen, a second assay was performed utilizing the chromogenic substrate S-2251 (Helena Labs). The activity of melanoma t-PA, the recombinant protein and bovine trypsin were first determined in the S-2288 assay and the concentrations were adjusted such that each enzyme was present at 100 units/100 $\mu$l. One hundred $\mu$l of melanoma t-PA, recombinant protein or bovine trypsin was then added in serial dilution to 100 $\mu$l of human plasminogen (0.15 mg/ml), and 800 $\mu$l of S-2251 substrate. The samples were incubated for 60 min at 37°C. The reaction was terminated by the addition of 1 ml of 50 percent acetic acid and absorbance at 405 nm was determined.

RESULTS

Electroporation of the construct pSVD8T (Fig. 1) into the 59D8 heavy chain loss variants provided numerous transfected clones. When approximately $1 \times 10^8$ hybridoma cells were mixed with 50 of circular plasmid DNA in 0.8 ml of phosphate buffered saline and subjected to a discharge of 2000 volts, approximately 15 $\mu$g of the wells on a 96-well plate contained drug-resistant clones. Approximately 75 percent of these clones were shown to secrete the recombinant protein. Five clones were chosen for further analysis on the basis of their growth rate and expression of mRNA coding for the fusion protein.

Western blot analysis of the affinity-purified recombinant protein were done. Blots of reduced gels probed with an iodinated anti-human t-PA monoclonal antibody revealed labeling of a 65-kD peptide. This is the expected size of a heavy chain-t-PA fusion protein. The β chain of t-PA is approximately 33 kD and the truncated heavy chain should contribute 30 kD. Several lines of evidence indicate that the 65 kD peptide is not a t-PA-like molecule contributed by fetal calf serum. The 65-kD band is observed when the transfected cell lines are grown in serum-free medium or in the intra-peritoneal space of mice. Also, when we purified bovine t-PA from fetal calf serum by benzamidine affinity chromatography, even though it was labeled by the antibody on Western blots, the size of the molecule was 75 kD.

Western blots of reduced samples probed with a goat anti-mouse Fab derived from polyclonal sera reveal labeling of a 25-kD protein, which is the expected size of the 59D8 κ light chain. Although on such blots this reagent usually labels the mouse immunoglobulin heavy chains also, the absence of labeling of the fusion peptide is not surprising since most of heavy chain constant region has been removed. Blots produced with unreduced samples show labeling of a single band at a molecular weight of 170-180 kD by both of the iodinated antibodies. This provides strong evidence that the hybridoma cells are producing a molecule containing both immunoglobulin and t-PA peptides. The 170-180 kD value suggests that the inter-heavy-chain disulfide bonds have formed to give a Fab'$_2$-like molecule that contains two antigen combining sites and two t-PA moieties.

The peptidolytic activity of the t-PA portion of the molecule was initially assessed by measuring the cleav-

EP 0 478 366 A2

age of the nonspecific substrate S-2288. Cleavage of this tripeptide can be accurately monitored by following the production of paranitroaniline, which absorbs light at a wavelength of 405 nm. Fig. 2A shows a typical assay, which employs directly the activity of differing concentrations of pure melanoma t-PA. Activity in this assay is defined as the rate of change in optical density. When a comparison is made on a molar basis between the recombinant protein and native t-PA, the recombinant protein possesses 70 percent of the activity of native t-PA.

To determine whether the catalytic β subunit maintained activity against plasminogen (its physiologic substrate), an S-2251 assay was performed. Here the plasminogen activator is required to convert plasminogen to plasmin and the plasmin subsequently liberates paranitroaniline from a synthetic tripeptide. Neither plasminogen activator nor trypsin can directly convert the S-2251 substrate. The amidolytic activities of the recombinant protein, melanoma t-PA and trypsin were first determined in the S-2288 assay, and then the ability of comparable amounts of each to convert plasminogen was determined. Fig. 2B reveals that the ability of the recombinant protein to act upon the physiologic substrate is very similar to that of native t-PA. Although a nonspecific serine protease such as trypsin is able to convert plasminogen to plasmin, it does so much less efficiently than does either the native or recombinant plasminogen activator.

Both the purification scheme and the assays used to following purification required an intact and functional antigen combining site. In order to more quantitatively compare the recombinant molecule with antibody 59D8, we employed a simple competition assay. This assay measured the ability of soluble fibrin monomer to compete for antibody binding sites against fibrin bound to the bottom of a 96-well plate. Although the assay indicates that the native antibody binds fibrin monomer better than does the recombinant protein, the difference in their binding affinities is less than 10-fold (Fig. 3). It is evident that antibody binding is not significantly impaired in the fusion protein.

DISCUSSION

Extensive analysis of the secreted protein indicates that a 59D8 heavy chain-t-PA fusion protein is being expressed and secreted in association with light chain in the manner predicted. The amount of recombinant protein present in cell culture supernatants, however, appears to be only 10 percent of that expected for monoclonal antibodies. By affinity purification, we routinely obtained only 0.1 μg of purified protein per milliliter of cell culture supernatant or 10 μg per ml in ascites. We monitored the purification with solid-phase immunoassays as described above, and our recoveries from the affinity columns were within the expected range. There are a number of possible reasons for the limited production of recombinant protein. One is that the recombinant protein is being degraded during cell growth or protein purification. In an attempt to limit proteolytic degradation, we have added protease inhibitors to the cell cultures. Although no improvement in yield was observed, proteolytic degradation remains a concern.

Other more fundamental problems could be the cause of the low yields of protein. Although messenger RNA of the appropriate size can be seen on Northern blot, transcription of the construct may occur at a low level. Transcription is driven by the natural heavy chain promoter and enhancer, but 3′ sequences, which have been shown to be important in regulation of immunoglobulin expression, have been excluded from this construct. Gregor et al., Mol. Cell. Biol. 6:1903-1916 (1986); Kobrin et al., Mol. Cell. Biol. 6:1687-1697 (1986). In addition, the 3′ untranslated region of the chimeric gene is from t-PA, a protein that is produced at a low level under normal conditions, and is subsequently stored in the cells where it is produced. It is possible that the 3′ UT region of the t-PA gene leads to low levels of transcription or translation, or interferes with secretion of the recombinant protein from the cell. Experiments aimed at quantitation of mRNA synthesis, protein synthesis, and stability of the recombinant peptide should allow resolution of this problem.

Heavy chain loss variants provide a convenient tool for the reconstitution of the antibody combining site. Their availability makes it unnecessary to clone and transfect the productive light chain rearrangement. This approach, of course, depends on being able to transfect these variant cell lines. The two lines used in these experiments were easily transfected using standard techniques, but it is not yet clear whether other SP2/0-derived lines will behave similarly. The amount of light chain that heavy chain loss variants secrete varies. However, some loss variants that secrete small quantities of light chain may be capable of secreting normal amounts of this same light chain when heavy chain synthesis is resumed. Wilde et al., Eur. J. Immunol. 10:462-067 (1980). Little is known about the biological basis for loss of immunoglobulin chain production in these cells and it is possible that the ability of some loss variants to produce light chain as well as heavy chain may be impaired. Our recombinant protein's low level of production could be the result of depressed light chain expression.

The recombinant t-PA β chain has a high level of catalytic activity, and it retains the specific ability to convert plasminogen to plasmin. Earlier studies, which linked staphylococcal nuclease and E. coli DNA polymerase functions to immunoglobulin heavy chain yielded considerably less effector function activity than the 70 percent

16

measured in the S-2288 assay. Neuberger et al., Nature 312:604-608 (1984); Williams et al., Gene 43:319-324 (1986). This retention of enzymatic activity and substrate specificity indicate that even complex molecules requiring strict folding and formation of multiple intrachain disulfide bonds can be used to form hybrid recombinant proteins. Others have shown that the β chain of t-PA is capable of folding correctly and maintaining activity in the absence of the β chain. MacDonald et al., Gene 42:59-67 (1986); von Zonneveld et al., Proc. Nat'l Acad. Sci. USA 83:4670-4674 (1986). Our results confirm the activity of the catalytic chain alone, and indicate that the chain can fold correctly in the context of a different amino terminal sequence. Together, these observations provide evidence for the independent folding of different protein domains.

In summary, we have cloned the heavy chain gene coding for the antigen combining site of an antifibrin antibody and produced a construct that codes for a truncated heavy chain-t-PA β subunit fusion peptide. The construct was subsequently transfected into heavy chain loss variants of the antifibrin hybridoma. Western blot analysis indicates that the fusion protein has antifibrin antibody activity and retains a level of plasminogen activating activity high enough to be considered similar to that of native t-PA.

## EXAMPLE 3

### Materials and Methods

Materials. The gene encoding the heavy chain of antifibrin monoclonal antibody 59D8 was cloned from high molecular weight genomic DNA isolated from 59D8 hybridoma cells as previously described (Quertermous, T. et al., J. Immunol. 138:2687 (1987); Schnee, J.M. et al., Proc. Natl. Acad. Sci. USA 84:6904 (1987)). The tPA sequence was obtained from a cDNA clone (pPA34'F) that had been constructed from HeLa cell mRNA (Fisher, R. et al., J. Biol. Chem. 260:11223 (1985)). Genomic UK DNA was a gift from F. Blasi. The 3′ portion of the human β-globin gene (Lawn, R.M. et al., Cell 21:647 (1980)), cloned in plasmid HβG1-D, was obtained from Dr. T. Maniatis. Final constructs were assembled in the pSV2gpt vector that had been modified by R. I. Near to include a polylinker containing a 6-kb Xba 1 restriction fragment encoding the murine λ2b heavy chain constant region (Mulligan, R.C. et al., Proc. Natl. Acad. Sci. USA 78:2072 (1981); Tucker, P.W. et al., Science 206:1303 (1979)).

Synthetic oligonucleotides were purchased from Synthetic Genetics, San Diego, CA. Reaction conditions and buffers for restriction enzymes, T4 DNA ligase, and the Klenow fragment of DNA polymerase 1 were those listed by the supplier, New England Biolabs (Beverly, MA). Transformation of Escherichia coli MC1061, preparation of plasmid DNAs, isolation of DNA fragments, and other standard recombinant techniques were carried out as described (Asusbel, F.M. et al., Current Protocols in Molecular biology, Greene Publishing Associates and Wiley-Interscience, New York (1989)).

Expression Vector Construction. The assembly of pSVtPA(tPA) has been described (Schnee, J.M. et al., Proc. Natl. Acad. Sci. USA 84:6904 (1987)). pSVtPA(Ig) was assembled by first isolating and ligating a 3.3-kb Sac I to Sal I fragment of the λ2b heavy chain gene (containing the 3′ untranslated region) inta pGEM3. A synthetic oligonucleotide encoding RI-XhoI-BamHI-Sac I restriction sites was inserted 5′ of the 3.3-kb λ2b fragment. Then a Bgl II to Bgl II 2.0-kb tPA cDNA fragment encoding the complete 527 amino acid sequence of tPA was ligated into the BamHI site of the synthetic polylinker. The tPA and λ2b fragments were isolated from the pGEM plasmid as a single fragment by Xho I and Sal I digestion. The Xho I to Sal I tPA encoding fragment of pSVtPA(tPA) was then removed to allow insertion of the 5.5-kb tPA-λ2b 3′ untranslated fragment and generation of the completed pSVtPA(Ig) plasmid.

The expression plasmid pSVUKG(UK) was constructed from a human urokinase gene. Axons coding for 32-kD single-chain urokinase, from amino acid 144, were assembled in pGEM3 that had been modified to contain an Xhol site. Coding sequence upstream of the EcoR1 site in Exon VII was constructed from complementary synthetic oligonucleotides. An Xho1 site was incorporated into the 5′ end of this sequence. An internal EcoR1 genomic fragment (1.3 kb) and a 3′ EcoR1-Sma1 fragment (3.4 kb) were assembled into the pGEM3 vector containing the 5′ sequence. An Xho1-Sal1 fragment containing the reconstructed urokinase genomic sequence was then inserted into the pSVtPA(tPA) construct (Fisher et al., J. Biol. Chem. 260:11223 (1985)), replacing the tPA sequence.

To generate pSVUKG(β), a synthetic oligonucleotide containing EcoR1-Xho1-BamH1-BglII-Sal1-HindIII sites was inserted into pUC19, creating pUC19M. The 5.0-kb Xho1 to Sal1 fragment from pSVUKG(UK) containing the single-chain urokinase gene was inserted into this modified pUC19 plasmid. A synthetic oligonucleotide was made which contained a 5′ BamH1 site, the urokinase sequence 3′ of the BamH1 site of Exon XI to the termination codon, and Bg12-Sal1 sites on its 3′ end. This oligonucleotide was used to replace the BamH1 to Sal1 fragment from the urokinase-containing pUC19M plasmid above (removing the urokinase 3′ untranslated domain). The human β-globin 3′ untranslated sequence was removed from pLL10, Rothstein, R.J., Lau, L.F., Bahl, C.P., Narang, S.A., and R. Wu. (1979) Synthetic Adapters for Cloning DNA. Methods in Enz. 68:98-109, by BamH1 digestion and ligated into the Bg12 site of pUC19M. Then, the β-globin sequence was removed from this pUC19 vector by BamH1 and Sal1 digestion and ligated into the pUC19M plasmid already containing the single-chain urokinase gene. Finally, the Xho1 to Sal1 fragment from this pUC19M vector (containing the urokinase gene beginning at Exon VII and a β-globin 3′ untranslated element) was substituted for the Xho1 to Sal1 fragment in pSVUKG(UK) to generate pSVUKG(β).

pSVUKG(Ig) was constructed by blunt end ligation of the 3.2 Sac1 to Sal1 λ2b 3′ untranslated sequence from pSV2gpt (see materials) into the Bgl2-Sal1 sites of the pUC19M vector described above which already contained the desired urokinase protein encoding sequence and termination codon. Then, the Xho1 to Sal1 fragment from this plasmid was used to replace the Xho1 to Sal1 fragment of pSVUKG(UK) to generate pSVUKG(IG).

Transfection and Selection. The isolation of heavy chain loss variants from the parental 59D8 cell line has been described (Schnee, J.M. et al., Proc. Natl. Acad. Sci. USA 84:6904 (1987)). Expression vectors (Figure 4) were linearized by Sal1 digestion and transfected by electroporation. A detailed description of the transfection and selection protocols has recently been published (Love, T.W. et al., Methods of Enzymology 178:515-527 (1989)). Essentially, loss variant cells were grown to half confluence, spun, and resuspended in 1 ml of buffer containing 20-100 µg/ml plasmid DNA. The cells were exposed to a single pulse of 200 volts (960 µFD) from a Gene Pulser electroporation apparatus (Bio-Rad, [Richmond, CA). Cells were then transferred to growth medium in the absence of mycophenolic acid.

Selection for clones transfected with the E. coli hypoxanthine guanine phosphoribosyltransferase gene (gpt) was begun 3 days after electroporation by exposure to medium containing mycophenolic acid (0.5 µg/ml), xanthine (100 µg/ml), and hypoxanthine (15 µg/ml).

Protein Concentration Determinations. Cells were allowed to grow to maximal confluence in 100-mm petri dishes (for approximately 48-72 hours) before harvesting of conditioned medium. Microtiter plates were coated with 25 µl of fibrin monomer solution (0.01 mg/ml). Conditioned media from each transfected cell lines was centrifuged, filtered, then serially diluted with phosphate-buffered saline (10 mM potassium phosphate, 0.15 M NaCl, pH 7.4). After the plates had been washed with distilled water, the wells were blocked by incubation for at least an hour with 50 µl of a 10% horse serum solution (to prevent nonspecific protein binding). The wells were rinsed again with distilled water, and 25 µl of each conditioned medium dilution was incubated for 2 hours. The cell medium was rinsed from the plates, and 25 µl of $^{125}$I-labeled goat anti-(mouse Fab) (50,000 cpm/25 µl) was added and allowed to incubate for 1 hour. The concentration of recombinant protein (assumed to be equal to functional 59D8 activity) was determined relative to a standard curve generated from measurements obtained with purified 59D8 antibody.

RNA isolation and analysis. RNA was prepared by conventional methods (14) from 59D8 myelomas, 59D8 heavy-chain loss variants, and each of the five transfected cell lines. RNA samples (10 µg/lane) were electrophoresed on a formaldehyde/agarose gel and transferred to nitrocellulose filters. The filters were hybridized with a $^{32}$P-labeled DNA probe that binds specifically to the 59D8 VDJ region. The VDJ probe consisted of a cloned 2.1-kb genomic DNA fragment spanning the VDJ exon. We confirmed equal loading of sample RNAs into lanes by probing blots with an actin probe. Actin mRNA was detected using a 700 basepair Pst1 fragment of pAct-1 plasmid (Spiegelman BM, M Frank, H. Green. 1983. Molecular cloning of mRNA from 3T3 adipocytes. J Biol. Chem. 258:10083.) provided by Lloyd Klickstein, Massachusetts General Hospital, Boston, Massachusetts.

Nuclear Runoff Transcription Analysis. The rate of transcription of the pSVUKG(UK) gene relative to that of the endogenous gene in the 59D8 hybridoma was measured essentially as described by Zagardo et al. (Shafit-Zagardo, B. et al., Nature 304:277 (1983)). Nuclei were harvested from both cell lines and incubated in $^{32}$P-labeled uridine triphosphates to label nascent RNA transcripts. The radiolabeled RNA was purified and hybridized to equal amounts of Eco R1-digested pSVUKG(UK) plasmid DNA that had been separated on an agarose gel and transferred onto nitrocellulose. Before hybridization, the number of counts/minute incorporated into each sample was determined to allow normalization for variable label in each hybridization. A LKB Ultrascan XL Laser Densitometer was used to determine the relative intensity of the hybridization signals.


RESULTS


Protein Expression Levels. The expression plasmids shown in Figure 4 were transfected into heavy chain loss variant 59D8 cells. Five unique, transfected cell lines were established, each secreting its encoded fusion protein. To measure the level of 59D8 antibody activity (and thus fusion protein secretion), we analyzed the conditioned medium from each transfected cell line with the radioimmunoassay described in Materials and Methods. Table 1 contains measurements of protein secretion for each of the cell lines. The parental 59D8 hybridoma secreted 7.6-10 μg/ml of functional 59D8 antibody, while the cell line pSVtPA(tPA) secreted a maximum of 0.025 μg/ml fusion protein. However, pSVtPA(Ig), which was modified from pSVtPA(tPA) to contain an Ig 3' untranslated domain, secreted as much as 2.5 μg/ml. This represents an approximate increase in protein secretion by 100-fold.

Similarly, pSVUKG(UK) secreted very low levels of fusion protein (0.015-0.06 μg/ml) compared to pSVUKG(Ig) and pSVUKG(β) (1-4.1 μg/ml). This corresponds to enhancements in protein secretion by 16- to 68-fold. The increases in protein expression produced by substitution of either the β-globin or λ2b 3' untranslated elements were approximately equal.

RNA Transfer Blot and Nuclear Run-off Analysis. RNA transfer blot analysis was performed using equal amounts of total cellular RNA from 59D8 hybridomas, heavy chain loss variants, and each of the five transfected cell lines. Equal loading of lanes was confirmed using an actin probe as described. In Figure 5 (panel A), the quantity of mRNA encoding 59D8 fusion protein in pSVtPA(tPA) and pSVUKG(UK) cells is confirmed to be dramatically lower than the amount of 59D8-encoding mRNA in the 59D8 myeloma cells. In the lane containing pSVtPA(tPA), no detectable message is visible. In pSVUKG(UK) there is a faint message corresponding to the predicted size of 2.7-KB.

Panel B of figure 5 contains a similar RNA transfer blot comparing mRNA levels in pSVtPA(Ig), pSVUKG(Ig), and pSVUKG(β) to 59D8 parental cells. In these transfectants, now containing genes with an immunoglobulin or β-globin 3' untranslated domain, the band intensities suggest mRNA levels which are more comparable to 59D8 cells. As shown in Table 1, the increased levels of mRNA in pSVtPA(Ig), pSVUKG(Ig), and pSVUKG(β) compared with pSVtPA(tPA) and pSVUKG(UK) is associated with a corresponding increase in protein secretion.

To define if the reduced levels of mRNA observed in the pSVUKG(UK) cell line compared to the native 59D8 hybridoma resulted from a lower rate of gene transcription, nuclear run-off transcription analysis was performed. As described above, the rate of mRNA transcription of each gene was accessed by probing a Southern blot containing EcoR-I digested pSVUKG(UK) plasmid DNA. The relative intensities of bands corresponding to DNA fragments common to pSVUKH(UK) and 59D8 were measured using a LKB Ultrascan XL Laser densitometer to determine the relative rates immunoglobulin heavy chain gene transcription. Table 2 contains relative values representing density of bands (after normalization for variable cpm used to hybridize) resulting from radiolabeled mRNA transcripts annealing to the 2.5-Kb VDJ exon fragment and the 1.9-Kb DNA fragment containing the heavy chain constant region sequence. The blots were exposed for 72 and 168 hour periods. The VDJ region bands yielded by mRNA isolated from pSVUKG(UK) nuclei were consistently more dense than those produced from mRNA purified from 59D8. This was also true of bands corresponding to the constant region sequences (despite the fact that 59D8 is a λ1 antibody). Although, we suspect the greater density of pSVUKG(UK) bands compared to corresponding 59D8 bands in within the error of the technique, this data suggest the rate of transcription in the transfected pSVUKG(UK) gene is at least as great as than of the endogenous 59D8 gene in the parental cell line. Thus, it appears unlikely that the differences in mRNA levels observed in pSVtPAB(tPA) and pSVUKG(UK) transfectants compared to pSVtPA(Ig), pSVUKG(Ig), and pSVUKG(β) relates to augmentation of gene transcription rates.

## DISCUSSION

The technique of creating novel proteins by transfection of recombinant genes into cells is becoming increasingly important (Morrison, S.L. et al., Adv. Immunol. 44:65 (1989)). Once incorporated into the genome of an appropriate cell line, a transfected gene can be transcribed and translated and the protein product assembled, processed, and secreted. Lymphoid cells have proven ideal recipients for transfection of immunoglobulin or immunoglobulin fusion protein genes (Morrison, S.L. et al., Adv. Immunol. 44:65 (1989); Neuberger, M.S. et al., Nature 312:604 (1984); Dorai, H. et al., J. Immunol. 139:4232 (1987)). unfortunately, a major limitation in the application of this technology has been low levels of protein expression from transfected genes (Morrison, S.L. et al., Adv. Immunol. 44:65 (1989); Dorai, H. et al., J. Immunol. 139:4232 (1987)). It has been suggested that transfectomas secrete poorly relative to hybridomas because these cells lack sufficient quantities of the necessary transcriptional factors (Maeda, H. et al., Cell 45:25 (1986); Sen, R. et al., Cell 46:705 (1986); Singh, H. et al., Nature 319:154 (1986)).

Our approach to resolving the problem of low protein expression of transfected genes involves taking advantage of the role 3' untranslated sequences play in determining mRNA stability. (Kabnick, K.S. et al., Mol. Cell. Bio. 8:3244 (1988); Purvis, I.J. et al., Nucleic Acids Res. 15:7951 (1988); Müllner, E.W. et al., Cell 53:815 (1988); Shaw, G. et al., Cell 46:659 (1986); Gregor, P.D. et al., Immunol. Reviews 89:31 (1986)). Kabnick and Housman have previously described prolongation of short-lived mRNA transcripts by substitution of a β-globin 3' untranslated element (Kabnick, K.S. et al., Mol. Cell. bio. 8:3244 (1988)). In this text, we describe the successful substitution of the human β-globin or the mouse λ2b immunoglobulin 3′ untranslated domains in transfected genes to increase both the levels mRNA and protein secretion from genes transfected into hybridomas.

Stable transfected cell lines were established for each of the genes shown in Figure 4, and the level of protein secretion for each cell line was determined (Table 1). RNA transfer blots were analyzed to determine if transfectants which secreted lower levels of protein also contained similarly diminished levels of mRNA. After establishing that low levels of protein secretion did correlate with low mRNA levels, nuclear run-off analysis demonstrated that there was not a significant difference in the rates of transcription of the transfected pSVUKG(UK) gene (which produced low levels of mRNA) and the endogenous 59D8 heavy chain gene (which produced high levels of mRNA). While we have not directly determined mRNA half-lives for each of the transfected genes, the mechanism of increased mRNA levels in the genes modified to contain an immunoglobulin or β-globin 3′ untranslated element appears most likely to relate to increased mRNA stability.

The exact mechanisms by which 3′ untranslated domains contribute to mRNA stability have not been defined. Proposed mechanisms have included the suggestion that a specific AU-rich 3′ untranslated sequence can promote mRNA degradation (Shaw, G. et al., Cell 46:659 (1986)). Others have proposed that mRNA 3′ untranslated regions can form secondary structures which might mediate mRNA stability by impeding access to exonucleases (Müllner, E.W. et al., Cell 53:815 (1988); Shaw, G. et al., Cell 46:659 (1986); Gregor, P.D. et al., Immunol. Reviews 89:31 (1986); Freier, S.M. et al., Proc. Natl. Acad. Sci. USA 83:9373 (1986); Zucker, M. et al., Nucleic Acids Res. 9:133 (1981)). Analysis of the human β-globin and mouse λ2b 3′ untranslated sequences using computer models do suggest that both sequences can potentially form stable loop structures (Zucker, M. et al., Nucleic Acids Res. 9:133 (1981)) (data not shown).

Despite the presence of 3′ untranslated elements from the human β-globin or mouse Ig genes, transfectomas [pSVtPA(Ig), pSVUKG(Ig), and pSVUKG(β)] never produced mRNA or protein levels equal to the original hybridoma. This suggest that factors other than the 3′ untranslated element are involved in determination of mRNA and protein levels. However, these experiments do indicate that both mRNA and protein levels may be increased in transfectants by modification of the 3′ untranslated element in the transfected gene. It is interesting to speculate if simultaneous 3′ untranslated element modification and DHFR-amplification (Dorai, H. et al., J. Immunol. 139:4232 (1987)) might achieve transfectomas capable of secreting protein levels exceeding the original hybridomas.

TABLE 1

| CELL LINE | PROTEIN LEVEL (ug/ml) | INCREASE |
|---|---|---|
| 59D8 | 7.6-10 | --- |
| pSVtPA(tPA) | 0.008-0.025 | --- |
| pSVtPA(Ig) | 0.25-2.5 | 100x |
| pSVUKG(UK) | 0.015-0.06 | --- |
| pSVUKG(Ig) | 1.0-4.05 | 68x |
| pSVUKG(β) | 1.0-4.1 | 68x |

TABLE 2

Relative transcription rates of the transfected pSVUKG(UK) and
endogenous 59D8 heavy chain genes.

|  | 59D8 | | pSVUKG(UK) | |
| --- | --- | --- | --- | --- |
|  | 72hr | 168hr | 72hr | 168hr |
| 2.5-kb VDJ Region | 0.6 | 1.5 | 1.9 | 3.7 |
| 1.9-Kb Heavy chain constant region | 0.3 | 0.4 | 0.6 | 1.5 |

EXAMPLE 4

A Recombinant Chimeric Plasminogen Activator with High Affinity for Fibrin with Increased Thrombolytic
Potency in vitro and in vivo

A genomic sequence coding for a portion of the mouse immunoglobulin γ2b constant region, genomic DNA
for the rearranged heavy chain gene of antibody 59D8, and a cDNA coding for the catalytic light chain of human
tissue plasminogen activator (tPA) was previously cloned into plasmid pSV2gpt (Schnee, J.M., et al., Proc. Natl.
Acad. Sci. USA 84:6904 (1987); Love, T.W., et al., In Methods in Enzymology, Langone, J.J. (ed.), Academic
Press, New york, pp. 515-527 (1989)). The resulting expression plasmid, pSVD8Tβ, was used as a starting
point for the constructs described here (Figure 6A). scuPA genomic DNA encoding amino acids 144 to 411
was then inserted into pSVD8Tβ in place of the tPA light chain gene (a plasmid containing genomic sequence
encoding the entire scuPA molecule was a gift from F. Blasi). An additional portion of the mouse immunoglobulin
γ2b constant region, containing sequence coding for the entire CH2 domain and a portion of the CH3 domain,
was also ligated into pSVD8Tβ. The resulting expression plasmid, pSVUKG(UK) (Figure 6A), was transfected
into 59D8 heavy-chain loss variant hybridoma cells by electroporation, as described Schnee, J.M., et al., Proc.
Natl. Acad. Sci. USA 84:6904 (1987); Love, T.W., et al., In Methods in Enzymology, Langone, J.J. (ed.),
Academic Press, New York, pp. 515-527 (1989)), and recombinant protein was purified from the culture supernatant by affinity chromatography.

Although initial assays of the culture supernatant indicated the presence of scuPA(32) and 59D8 antigens,
and both activities, the levels of expressed r-scuPA(32)-59D8 protein was extraordinarily low (Figure 6B), on
the order of 30 to 200 ng/ml of culture supernatant. Northern blot analysis with poly A+RNA from P220R-15
cells [a stable, subcloned line expressing pSVUKG(UK)] that had been probed with two $^{32}$P-labeled oligonuc-
leotides (one specific for mouse IgG, the other specific for scuPA) showed a low steady-state mRNA level (relative to that of antibody 59D8 in control cells) consisting of a single, 2.7-kb transcript (not shown). Nuclear run-off
experiments indicated that this low steady-state mRNA level was likely due to mRNA instability rather than to
a reduced transcription rate (not shown). We then replaced the 3′ untranslated (UT) domain [that of scuPA in
pSVUKG(UK)] with the 3′ UT domain of β globin (pSVUKG(β), Figure 6A] or immunoglobulin [pSVUKG(Ig), Figure 6A]. This change produced a better than 100-fold improvement in levels of protein expression (Figure 6B),
with corresponding increases in steady-state mRNA levels (not shown). The protein expression level was similarly greater for the plasmid pSVUKc(Ig) (Figure 6A), in which the genomic DNA encoding amino acids 144 to
411 of scuPA had been replaced with cDNA encoding the same sequence and the plasminogen activator 3′
UT domain had been replaced with the immunoglobulin 3′ UT domain (not shown). The r-scuPA(32)-59D8 protein tested in the in vitro and in vivo studies that follow came from the transfection (by electroporation) of the
pSVUKG(β) plasmid into 59D8 heavy-chain loss variant cells (L2LV).

Milligram quantities of r-scuPA(32)-59D8 protein were produced by growing cells to high density (total mass
of approximately 4 x 10[10] cells) in DMEM with 10% FCS in the extrafiber space of a CellMax (Type B) bioreactor
(Cellco Advanced Bioreactors, 5516 Nicholson Lane, Kensington, MD 20895) containing cellulose acetate hol-

21

low fibers with a sieving coefficient of approximately 4 kDa. Culture medium was harvested at 12-hour intervals and immediately frozen at -70°C. After the aliquots had been thawed and pooled, r-scuPA(32)-59D8 was purified from the medium (the initial concentration of recombinant protein varied between 0.05 and 0.3 mg/ml) by affinity chromatography on a resin containing Sepharose linked to the heptapeptide epitope for antibody 59D8 (Runge, M.S., et al., Biochemistry 27:1153 (1988)). The eluate contained a mixture of r-scuPA(32)-59D8 (15 to 35%) and enzymatically cleaved r-scuPA(32)-59D8 (85 to 65%). (Either thrombin or plasmin can cleave the scuPA portion of r-scuPA(32)-59D8 into low molecular weight two-chain urokinase.) r-scuPA(32)-59D8 was obtained by passing this mixture through a column of benzamidine-Sepharose. (Benzamidine-sepharose chromatography was performed as described (Runge, M.S., et al., Proc. Natl. Acad. Sci. USA 84:7659 (1987)). Rather than being used for affinity binding of the desired product (Haber, E., et al., Science 243:51 (1989)), benzamidine-Sepharose was used to remove two-chain r-scuPA(32)-59D8 from the mixture of single- and two-chain forms.) Purified r-scuPA(32)-59D8 (0.05 tg 0.5 mg/ml in Tris-glycine buffer, pH 7.4) was used immediately in assays or frozen at -70°C, at which it remained stable for up to 6 weeks.

The predicted MW of purified r-scuPA(32)-59D8 is approximately 95 kDa: ~38 kDa for the truncated 59D8 "heavy chain" (lacking CH3), ~32 kDa for scuPA(32) (not accounting for glycosylation on either component molecule) and ~25 kDa for the 59D8 "light chain." Under nonreducing conditions, SDS-polyacrylamide gel electrophoresis of purified r-scuPA(32)-59D8 (Figure 7) revealed a single predominant band at ~104 kDa, which accounts for all the predicted components. The band contained immunoglobulin (Figure 7) and scuPA (not shown) epitopes by western blot analysis. The presence of a small amount of heavy chain-scuPA fusion peptide is evidenced by the band at 78 kDa (Figure 7). Under reducing conditions (Figure 7), major bands included the heavy chain-scuPA fusion peptide (78 kDa) and light chain (25 kDa) species; the two intermediate bands most likely resulted from degradation of the heavy chain-scuPA fusion peptide by thrombin (at $Arg^{156}$-$Phe^{157}$) or plasmin (at $Lys^{158}$-$Ile^{159}$). The identity of these bands was confirmed by western blot analysis (Figure 7).

To evaluate the functional properties of r-scuPA(32)-59D8, we compared the catalytic activity and $K_m$ for plasminogen of plasmin-cleaved r-scuPA(32)-59D8 with those of low molecular weight two-chain urokinase, and compared the fibrin-binding activity with that of native 59D8. The catalytic activity of tissue culture-derived scuPA was 85,000 IU/mg. This material was >95% uncleaved (i.e., single-chain) when latent activity was compared with activity after cleavage to high molecular weight two-chain urokinase [(scuPA and r-scuPA(32)-59D8 were converted to the two-chain form with plasmin-Sepharose (Runge, M.S., et al., Proc. Natl. Acad. Sci. USA 84:7659 (1987); Dewerchin, M., et al., Eur. J. Biochem. 185:141 (1989))]. Preparations of r-scuPA(32)-59D8 were 90% single chain, with a catalytic activity of 26,000 IU/mg of protein after conversion to the two-chain form. Given the contribution, on a molar basis, of the 32-kDa scuPA portion of the 104-kDa r-scuPA(32)-59D8 molecule, the activity of the scuPA portion of 83,900 IU/mg of scuPA. This is not significantly different from the activity of native scuPA. In addition, the $K_m$ (16.6 μM) of the plasmin-cleaved (two-chain) form of r-scuPA(32)-59D8 did not differ significantly from that of low molecular weight two-chain urokinase (9.1 μM) (Figure 8A). The fibrin-binding activity of r-scuPA(32)-59D8 did not differ significantly from that of low molecular weight two-chain urokinase (9.1 μM) (Figure 8A). The fibrin-binding activity of r-scuPA(32)-59D8 [native scuPA(32) does not bind fibrin directly] was compared with that of native 59D8 by measuring the binding of serial dilutions of either 59D8 or r-scuPA(32)-59D8 to fibrin monomer-coated, 96-well plates. The fibrin binding of both species was comparable within the error of the method, indicating that the fibrin-binding domain of r-scuPA(32)-59D8 did not differ significantly from that of native 59D8 (Figure 8B).

In a human plasma clot assay (Lijnen, H.R., et al., Thromb. Haemostasis 52:308 (1984)), r-scuPA(32)-59D8 was 6 times more potent than scuPA (p < 0.0001) (Figure 9A). The results were even more striking in vivo, when tested in the rabbit jugular vein model (the in situ formation of a human thrombus in the rabbit's vein) (Runge, M.S., et al., Proc. Natl. Acad. Sci. USA 84:7659 (1987): Collen, D., et al., Fibrinolysis 3:197 (1989)). Compared with scuPA, r-scuPA(32)-59D8 displayed a remarkable 20-fold increase in the thrombolytic potency in vivo over the entire dose-response range (p < 0.0001) (Figure 9B). r-scuPA(32)-59D8 did not cause a decrease in fibrinogen concentration until 83% lysis was reached, at which point the fibrinogen concentration was 79% that of the control.

r-scuPA(32)-59D8 demonstrates that it is possible to design a plasminogen activator dimer in which the activities of the components, though comparable to those of the native proteins, manifest increased selectivity and potency when combined in a single molecule. This approach promises to address two of the remaining problems in plasminogen activator therapy: bleeding and incomplete lysis.

## Claims

1. An immunoglobulin molecule comprising an antibody variable region with an antigen binding site specific for fibrin; and a fibrinolytic enzyme activity region.

2. An immunoglobulin molecule as claimed in claim 1 wherein the fibrinolytic enzyme is tissue-type plasminogen activator, streptokinase, urokinase, or prourokinase.

3. An immunoglobulin molecule as claimed in claim 1 or 2 wherein the fibrinolytic enzyme is single chain urokinase and/or wherein the antibody variable region is from antibody 59D8.

4. An immunoglobulin molecule as claimed in any of claims 1 to 3 which is r-scuPA(32)-59D8.

5. Nucleic acid encoding an immunoglobulin molecule as claimed in any of claims 1 to 4.

6. Nucleic acid as claimed in claim 5 which is a recombinant DNA molecule comprising a sequence encoding the heavy chain of antibody 59D8 linked with a sequence encoding single-chain urokinase, such as a molecule comprising the 3′ untranslated region of β-globin and/or the 3′ untranslated region of lambda2b heavy chain gene.

7. Nucleic acid as claimed in claim 5 or 6 which is a recombinant DNA molecule comprising pSVUKG(Ig) and/or pSVUKG(B).

8. A vector comprising nucleic acid according to any of claims 5 to 7.

9. A host comprising a vector according to claim 8.

10. A process for the preparation of an immunoglobulin molecule comprising an antibody variable region with an antigen binding site specific for fibrin, and a fibrinolytic enzyme activity region, the process comprising:
transforming a host with an expression vector according to claim 8;
growing the host such that nucleic acid is expressed; and
optionally purifying the immunoglobulin molecule.

11. A pharmaceutical composition comprising an immunoglobulin molecule according to any of claims 1 to 4 and a pharmaceutically acceptable carrier.

12. A method of detecting a thrombus the method comprising:
(a) administering to a host an immunoglobulin molecule according to any of claims 1 to 4 wherein the molecule is labelled with a detectable marker, such as a radiolabel and/or paramagnetic isotope; and
(b) detecting the presence of a thrombus.

13. An immunoglobulin molecule as claimed in any of claims 1 to 4 for use in medicine.

14. The use of an immunoglobulin molecule as claimed in any of claims 1 to 4 in the preparation of an agent for treating a thrombus and/or a thrombolytic, fibrinolytic or diagnostic agent.

### Claims for the following Contracting States : ES, GR

1. A process for the preparation of an immunoglobulin molecule comprising an antibody variable region with an antigen binding site specific for fibrin, and a fibrinolytic enzyme activity region, the process comprising:
transforming a host with an expression vector comprising nucleic acid encoding the immunoglobulin molecule;
expressing the nucleic acid to produce the immunoglobulin molecule; and
optionally purifying the immunoglobulin molecule.

2. A process as claimed in claim 1 wherein the fibrinolytic enzyme is tissue-type plasminogen activator, streptokinase, urokinase, or prourokinase.

3. A process as claimed in claim 1 or 2 wherein the fibrinolytic enzyme is single chain urokinase and/or wherein the antibody variable region is from antibody 59D8.

4. A process as claimed in any of claims 1 to 3 wherein the immunoglobulin molecule is r-scuPA(32)-59D8.

5. A process for the preparation of nucleic acid encoding an immunoglobulin molecule comprising an antibody variable region with an antigen binding site specific for fibrin, and a fibrinolytic enzyme activity region.

6. A process as claimed in claim 5 for preparing a recombinant DNA molecule comprising a sequence encoding the heavy chain of antibody 59D8 linked with a sequence encoding single-chain urokinase, such as a molecule comprising the 3′ untranslated region of β-globin and/or the 3′ untranslated region of lambda2b heavy chain gene.

7. A process as claimed in claim 5 or 6 for preparing a recombinant DNA molecule comprising pSVUKG(Ig) and/or pSVUKG(β).

8. A process for the preparing a vector comprising nucleic acid encoding an immunoglobulin molecule comprising an antibody variable region with an antigen binding site specific for fibrin, and a fibrinolytic enzyme activity region, the process comprising coupling successive nucleotides together.

9. A process for preparing a host, the process comprising transforming or transfecting a host cell with a vector prepared according to claim 8.

10. A process for the preparation of a pharmaceutical composition, the process comprising admixing an immunoglobulin molecule comprising an antibody variable region with an antigen binding site specific for fibrin, and a fibrinolytic enzyme activity region, and a pharmaceutically acceptable carrier.

11. A method of detecting a thrombus, the method comprising:
    (a) administering to a host an immunoglobulin molecule comprising an antibody variable region with an antigen binding site specific for fibrin, and a fibrinolytic enzyme activity region, wherein the molecule is labelled with a detectable marker, such as a radiolabel and/or paramagnetic isotope, and
    (b) detecting the presence of a thrombus.

12. The use of an immunoglobulin molecule comprising an antibody variable region with an antigen binding site specific for fibrin, and a fibrinolytic enzyme activity region, in the preparation of an agent for treating a thrombus and/or a thrombolytic, fibrinolytic or diagnostic agent.

FIGURE 1

FIGURE 2A

FIGURE 2B

FIGURE 3

_Concentration of soluble fibrin, μg_

# tPA Constructs    FIGURE 4

| VDJ | CH₁ | H | tPA(B)cDNA | tPA 3'UT | pSVtPA(tPA) |

| VDJ | CH₁ | H | tPA(AB)cDNA | Ig 3'UT | pSVtPA(Ig) |

# Urokinase Constructs

| VDJ | CH₁ | H | Urokinase | UK 3'UT | pSVUKG(UK) |

| VDJ | CH₁ | H | Urokinase | Ig 3'UT | pSVUKG(Ig) |

| VDJ | CH₁ | H | Urokinase | ß-globin 3'UT | pSVUKG(ß) |

## FIGURE 5A

A

## FIGURE 5B

B

FIGURE 6A

FIGURE 6B

FIGURE 7

FIGURE 8A

**1/V**

[Plasminogen (μM$^{-1}$)]

FIGURE 8B

FIGURE 9A

FIGURE 9B